(19) Europäisches Patentamt
European Patent Office
Office européen des brevets

(11) EP 3 998 254 A1

(12) **EUROPEAN PATENT APPLICATION**
published in accordance with Art. 153(4) EPC

(43) Date of publication:
**18.05.2022 Bulletin 2022/20**

(21) Application number: **20835339.1**

(22) Date of filing: **01.07.2020**

(51) International Patent Classification (IPC):
***C07D 239/48*** (2006.01) ***C07D 401/14*** (2006.01)
***C07D 413/12*** (2006.01) ***A61K 31/5377*** (2006.01)
***A61K 31/541*** (2006.01) ***A61K 31/506*** (2006.01)
***A61P 35/00*** (2006.01) ***A61P 35/02*** (2006.01)

(86) International application number:
**PCT/CN2020/099683**

(87) International publication number:
**WO 2021/000884 (07.01.2021 Gazette 2021/01)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **04.07.2019 CN 201910598383**

(71) Applicant: **Shenyang Pharmaceutical University**
**Shenyang**
**Liaoning 110016 (CN)**

(72) Inventors:
- **ZHAO, Yanfang**
  **Shenyang, Liaoning 110016 (CN)**
- **LI, Yingxiu**
  **Shenyang, Liaoning 110016 (CN)**
- **QIN, Mingze**
  **Shenyang, Liaoning 110016 (CN)**
- **HOU, Yunlei**
  **Shenyang, Liaoning 110016 (CN)**
- **LIU, Yajing**
  **Shenyang, Liaoning 110016 (CN)**
- **GONG, Ping**
  **Shenyang, Liaoning 110016 (CN)**

(74) Representative: **Altmann Stößel Dick**
**Patentanwälte PartG mbB**
**Theodor-Heuss-Anlage 2**
**68165 Mannheim (DE)**

(54) **2-AMINOPYRIMIDINE COMPOUND AND APPLICATION THEREOF**

(57) The present invention relates to 2-aminopyrimidine compounds of general formula I and pharmaceutically acceptable salts, solvates or prodrugs thereof, their preparation methods and pharmaceutical compositions containing the compounds, wherein the substituents $R_1$ and $R_2$, $R_3$, $R_4$, X, Y, Z, Q, m, n have the meanings given in the specification. The present invention also relates to the use of the compounds of general formula I in the preparation of a medicament for the treatment and/or prevention of hematological malignancies and other proliferative diseases.

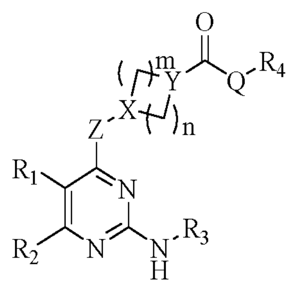



Processed by Luminess, 75001 PARIS (FR)

## Description

This application claims the priority of Chinese Patent Application No. 201910598383.X with the title of "2-Aminopyrimidine Compounds and Uses Thereof" filed on July 4, 2019, and the contents of which are incorporated herein in its entirety by reference.

## FIELD OF THE INVENTION

The present invention relates to the field of medicinal chemistry, in particular to a series of novel 2-aminopyrimidine compounds and their pharmaceutically acceptable salts, solvates or prodrugs, their preparation methods and pharmaceutical compositions containing the compounds. The present invention also relates to the use of such compounds in the preparation of medicaments for the treatment and/or prevention of hematological malignancies and other proliferative diseases.

## BACKGROUND OF THE INVENTION

Hematological malignancies are tumorous diseases of the blood system caused by disorders of the hematopoietic system, which mainly include leukemia, lymphoma, myelodysplastic syndrome and multiple myeloma etc. Due to the abnormal hematopoietic function in patients with hematological malignancies, combined with chemotherapy or radiotherapy which inhibits the patient's bone marrow hematopoietic and immune functions, the patients are prone to nosocomial infections, thus this will lead to aggravation of the condition and even death.

JAK kinase (janus kinase) is an intracellular non-receptor tyrosine protein kinase, and JAK2 is one of the important members of the JAK kinase family. JAK2 can form cell signal transduction pathways with multiple members of the STAT family, such as JAK2-STAT3 and JAK2-STAT5. Under normal physiological conditions, erythropoietin, interleukin, thrombopoietin and other cytokines bind to a receptor to cause homologous or heterologous oligomerization of the receptor, thereby activating the autophosphorylation of JAK2 kinase coupled to the receptor. The activated JAK2 catalyzes the phosphorylation of tyrosine residues on the receptor and forms a "docking site" with surrounding amino acids, allowing the STAT protein containing the SH2 domain to bind to it and be phosphorylated. Activated STATs can form homologous or heterologous dimers and quickly enter the nucleus to induce gene transcription. The JAK2-STATs signaling pathway is an important pathway for cell signal transduction, and it plays an important role in regulating the normal physiological and pathological responses of the human body. The abnormally elevated activity of JAK2 will make the JAK2-STATs signaling pathway be abnormally regulated, leading to the occurrence of various malignant diseases.

FLT3 is a member of the type III receptor tyrosine kinase family and plays an important role in the proliferation, differentiation and apoptosis of hematopoietic cells and lymphocytes. FLT3 gene mutations can cause abnormal activation of kinases, thus without relying on ligands, autophosphorylation of the kinases occurs, which activates a series of downstream signaling pathways, leading to abnormal proliferation of hematopoietic cells and lymphocytes, and triggering a variety of hematological malignancies. Therefore, inhibition of FLT3 kinase and its mutants is an effective method to treat related blood diseases, especially acute myeloid leukemia (AML).

The abnormally elevated activity of JAK2 and FLT3 kinases is closely related to the occurrence of hematological malignancies, and the medical needs of myelofibrosis (MF), lymphoma and AML in hematological malignancies have not been met. Therefore, the research of JAK2/FLT3 inhibitors has become a new field for the treatment of such diseases.

At present, there are few related literature reports on JAK2/FLT3 inhibitors, and there are no approved JAK2/FLT3 small molecule inhibitors. However, some compounds with outstanding activities and good therapeutic effects are already in the preclinical and clinical research stages, such as: Pacritinib ( J. Med. Chem., 54 (2011) 4638-4658), Fedratinib (Cancer Cell, 13 (2008) 311-320), Lestaurtinib (Blood, 103 (2004) 3669-3676), etc. Momelotinib reported in the literature is a 2-aminopyrimidine JAK1 and JAK2 kinase inhibitor developed by Gilead, with $IC_{50}$ of 11 nM and 18 nM, respectively. Momelotinib is currently in the clinical phase III research stage for the treatment of bone marrow fibrosis, polycythemia and thrombocytosis.

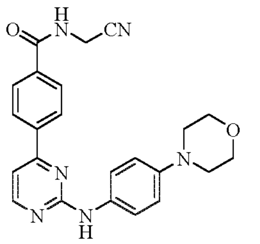

Momelotinib

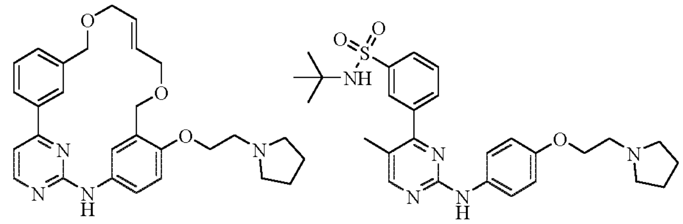

Pacritinib Fedratinib

The inventors designed and synthesized a series of 2-aminopyrimidine derivatives on the basis of references, these derivatives are tested in an assay in vitro for JAK2 and FLT3 kinase inhibitory activity, and the results showed that they all have inhibitory activities.

SUMMARY OF THE INVENTION

The present invention relates to 2-aminopyrimidine compounds of general formula I and their pharmaceutically acceptable salts, solvates or prodrugs thereof,

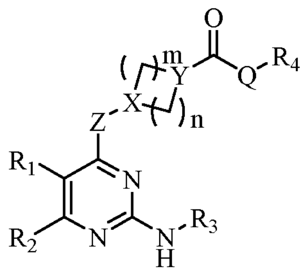

I

wherein, X and Y are the same or different, and are independently selected from N and CH;

Q is NH or $CH_2$;
Z is selected from the group consisting of NH, $NCH_3$, O or a chemical bond;
m and n are the same or different, and are integers between 1 to 3;
$R_1$ and $R_2$ are the same or different, and are independently selected from the group consisting of hydrogen, halogen, $(C_1\text{-}C_6)$alkyl, $(C_1\text{-}C_6)$alkoxy, trifluoromethyl, cyano, amino, and nitro, or $R_1$ and $R_2$ together form $(C_6\text{-}C_{10})$ aryl, 5 to 10 membered heteroaryl or 4-10 membered heterocyclic group, the heterocyclic group optionally includes 0 to 3 double bonds;
$R_3$ is $(C_1\text{-}C_6)$ alkylacyl, $(C_6\text{-}C_{10})$ aryl or 5 to 10 membered heteroaryl, the aryl or heteroaryl is optionally substituted by 1 to 3 same or different $R_8$ groups;
$R_8$ is hydroxy, halogen, cyano, $(C_1\text{-}C_6)$ alkyl, $(C_1\text{-}C_6)$ alkoxy, $(C_1\text{-}C_6)$ alkylacyl, $(C_1\text{-}C_6)$ carbamoyl, $-NR_5R_6$, $-(CH_2)_pNR_5R_6$, $-CONR_5R_6$, - $NHCONR_5R_6$, $-O(CH_2)_pNR_5R_6$, $-SO_2(CH_2)_pNR_5R_6$, or $-SO_2(CH_2)_pCONR_5R_6$;
wherein, $R_5$ and $R_6$ are the same or different, and are independently selected from the group consisting of hydrogen, $(C_1\text{-}C_6)$ alkyl, $(C_3\text{-}C_7)$ cycloalkyl, $(C_2\text{-}C_6)$ alkenyl, $(C_2\text{-}C_6)$ alkynyl, $(C_1\text{-}C_6)$ alkylacyl and $(C_1\text{-}C_6)$ alkoxy;
or $R_5$ and $R_6$ together with the nitrogen atom to which they are connected form a 4 to 10 membered heterocyclic group or a 5 to 10 membered heteroaryl, the heterocyclic group or heteroaryl except for the nitrogen atom connected to $R_5$ and $R_6$, optionally contains 0 to 4 heteroatoms selected from N, O and/or S, the heterocyclic group optionally includes 0 to 3 double bonds, and the heterocyclic group or heteroaryl is optionally substituted by 0 to 3 same or different $R_7$ groups;
$R_7$ is $(C_1\text{-}C_6)$alkyl or $(C_3\text{-}C_7)$cycloalkyl;
p is an integer of 0 to 4;
$R_4$ is $(C_1\text{-}C_6)$ alkyl, $(C_3\text{-}C_7)$ cycloalkyl, $(C_6\text{-}C_{10})$ aryl, 5 to 10 membered heteroaryl, $(C_6\text{-}C_{10})$ arylmethyl, 5 to 10 membered heteroarylmethyl, the aryl or heteroaryl is optionally substituted by 1 to 3 same or different $R_9$ groups;
$R_9$ is hydroxy, halogen, halogenated $(C_1\text{-}C_6)$ alkyl, halogenated $(C_1\text{-}C_6)$ alkoxy, nitro, amino, cyano, $(C_1\text{-}C_6)$ alkyl, $(C_2\text{-}C_6)$ alkenyl , $(C_2\text{-}C_6)$ alkynyl, $(C_1\text{-}C_6)$ alkoxy, $(C_1\text{-}C_6)$ alkyl or $(C_1\text{-}C_6)$ alkoxy optionally substituted by hydroxy,

amino or halogen, amino substituted by 1 to 2 $(C_1-C_6)$ alkyl, $(C_1-C_6)$ alkyl amide, free, salt-formed, esterified or amidated carboxyl, $(C_1-C_6)$ alkylsulfinyl, $(C_1-C_6)$ alkylsulfonyl, $(C_1-C_6)$ alkoxy, $(C_1-C_6)$ alkyl, $(C_1-C_6)$ alkylacyl, $(C_1-C_6)$ carbamoyl, carbamoyl substituted by 1 to 2 $(C_1-C_6)$ alkyl, $(C_1-C_3)$alkylenedioxy, or allyl.

In the present invention, the 2-aminopyrimidine compounds of the general formula I and their stereoisomers, pharmaceutically acceptable salts, solvates or prodrugs thereof according to claim 1 are preferred,

wherein, Z is selected from NH, $NCH_3$ or a chemical bond;
$R_1$ and $R_2$ are the same or different, and are independently selected from the group consisting of hydrogen, halogen, $(C_1-C_6)$alkyl, $(C_1-C_6)$alkoxy, trifluoromethyl, cyano, amino, nitro, or $R_1$ and $R_2$ together form $(C_6-C_{10})$ aryl, or 5 to 10 membered heteroaryl;
$R_3$ is $(C_6-C_{10})$ aryl, 5 to 10 membered heteroaryl, and the aryl or heteroaryl is optionally substituted with 1 to 3 same or different $R_8$ groups.

The present invention preferably also relates to the 2-aminopyrimidine compounds of the general formula I and their stereoisomers, pharmaceutically acceptable salts, solvates or prodrugs thereof,

wherein,
m and n are the same or different and are 1or 2;
$R_3$ is phenyl or 5 to 6 membered heteroaryl, and the phenyl or heteroaryl is optionally substituted with 1 to 3 same or different $R_8$ groups;
$R_8$ is $(C_1-C_6)$ alkoxy, $(C_1-C_6)$ alkylacyl, $(C_1-C_6)$ carbamoyl, $-NR_5R_6$, $-(CH_2)_pNR_5R_6$, $-CONR_5R_6$, $-NHCONR_5R_6$, $-O(CH_2)_pNR_5R_6$ or $-SO_2(CH_2)_pNR_5R_6$;
$R_5$ and $R_6$ are the same or different, and are independently selected from the group consisting of hydrogen, $(C_1-C_4)$alkyl, $(C_3-C_6)$cycloalkyl, and $(C_1-C_4)$alkylacyl;
or $R_5$ and $R_6$ together with the nitrogen atom to which they are connected form

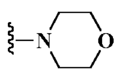 , 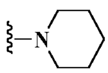 , 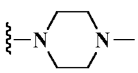 , 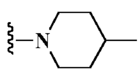 , 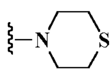 ,

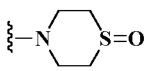 , 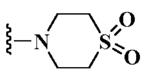 or 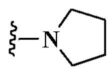 ;

$R_4$ is phenyl group, or 5-6 membered heteroaryl, and the phenyl or heteroaryl is optionally substituted with 1 to 3 same or different $R_9$ groups.

In the present invention, the 2-aminopyrimidine compounds of general formula I and their stereoisomers, pharmaceutically acceptable salts, solvates or prodrugs thereof are more preferable,

Z is NH or a chemical bond;
$R_1$ and $R_2$ are the same or different, and are independently selected from the group consisting of hydrogen, fluorine, chlorine, methyl, ethyl, cyclopropyl, methoxy, trifluoromethyl, cyano, amino, nitro, or $R_1$ and $R_2$ together form phenyl or 5 to 6 membered heteroaryl;
$R_8$ is $(C_1-C_6)$ alkoxy, $(C_1-C_6)$ alkylacyl, $(C_1-C_6)$ carbamoyl, $-NR_5R_6$, $-(CH_2)_pNR_5R_6$, $-CONR_5R_6$, $-NHCONR_5R_6$, or $-O(CH_2)_pNR_5R_6$;
or $R_5$ and $R_6$ together with the nitrogen atom to which they are connected form

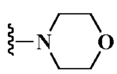 , 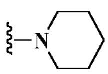 , 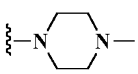 , 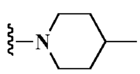 , 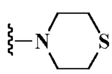 ,

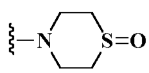 , 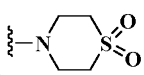 or 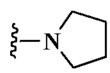 .

In the present invention, the 2-aminopyrimidine compounds of general formula I and their stereoisomers, pharmaceutically acceptable salts, solvates or prodrugs thereof are further preferred,

wherein,
Q is NH;
X and Y are N;
Z is a chemical bond;
m and n are 2;
$R_1$ and $R_2$ are the same or different, and are independently selected from the group consisting of hydrogen, fluorine, chlorine, methyl, methoxy, trifluoromethyl, amino, and nitro;
$R_3$ is phenyl optionally substituted with 1 to 3 same or different $R_8$ groups;
$R_8$ is $-NR_5R_6$, $-(CH_2)_pNR_5R_6$, $-CONR_5R_6$, or $-O(CH_2)_pNR_5R_6$;
p is an integer of 1 to 4;
$R_4$ is phenyl optionally substituted with 1 to 3 same or different $R_9$ groups.

The present invention particularly preferably relates to 2-aminopyrimidine compounds of general formula I and their stereoisomers, pharmaceutically acceptable salts, solvates or prodrugs thereof,

wherein,
$R_2$ is hydrogen;
$R_8$ is $-NR_5R_6$, $-O(CH_2)_pNR_5R_6$;
p is 2 or 3.

The present invention particularly preferably also relates to 2-aminopyrimidine compounds of general formula I and their stereoisomers, pharmaceutically acceptable salts, solvates or prodrugs thereof,

wherein,
$R_1$ is hydrogen, fluorine, chlorine, methyl or trifluoromethyl;
$R_5$ and $R_6$ together with the nitrogen atom to which they are connected form

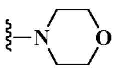 , 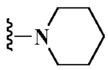 , 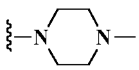 , 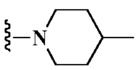 , 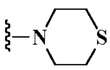 , 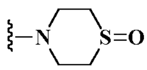 ,

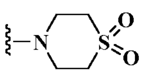 or 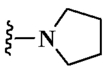 .

In the present invention, the following 2-aminopyrimidine compounds of general formula I and their stereoisomers, pharmaceutically acceptable salts, solvates or prodrugs thereof are particularly preferred:

*N*-(4-methoxyphenyl)-4-(2-{[4-(morpholin-4-yl)phenyl]amino}pyrimidin-4-yl)piperazine-1-carboxamide;
*N*-(4-trifluoromethoxyphenyl)-4-(2-{[4-(morpholin-4-yl)phenyl]amino}pyri midin-4-yl)piperazine-1-carboxamide;
*N*-(4-ethylphenyl)-4-(2-{[4-(morpholin-4-yl)phenyl]amino}pyrimidin-4-yl)piperazine-1-carboxamide;
*N*-phenyl-4-(2-{[4-(morpholin-4-yl)phenyl]amino}pyrimidin-4-yl)piperazine -1-carboxamide;
*N*-(4-methoxyformylphenyl)-4-(2-{[4-(morpholin-4-yl)phenyl]amino}pyrimi din-4-yl)piperazine-1-carboxamide;
*N*-(4-acetylphenyl)-4-(2-{[4-(morpholin-4-yl)phenyl]amino}pyrimidin-4-yl)piperazine-1-carboxamide;
*N*-(4-nitrophenyl)-4-(2-{[4-(morpholin-4-yl)phenyl]amino}pyrimidin-4-yl)piperazine-1-carboxamide;
*N*-(4-cyano-3-fluorophenyl)-4-(2-{[4-(morpholin-4-yl)phenyl]amino}pyrimi din-4-yl)piperazine-1-carboxamide;
*N*-(4-isopropoxyphenyl)-4-(2-{[4-(morpholin-4-yl)phenyl]amino}pyrimidin-4-yl)piperazine-1-carboxamide;
*N*-(4-carbamoylphenyl)-4-(2-{[4-(morpholin-4-yl)phenyl]amino}pyrimidin-4-yl)piperazine-1-carboxamide;
*N*-(4-methylcarbamoylphenyl)-4-(2-{[4-(morpholin-4-yl)phenyl]amino}pyri midin-4-yl)piperazine-1-carboxamide;
*N*-(4-aminosulfonylphenyl)-4-(2-{[4-(morpholin-4-yl)phenyl]amino}pyrimid in-4-yl)piperazine-1-carboxamide;
*N*-(4-acetylaminophenyl)-4-(2-{[4-(morpholin-4-yl)phenyl]amino}pyrimidin -4-yl)piperazine-1-carboxamide;
*N*-(4-methanesulfonylphenyl)-4-(2-{[4-(morpholin-4-yl)phenyl]amino}pyri midin-4-yl)piperazine-1-carboxamide;
*N*-(4-carbamoylphenyl)-4-(2-{[4-(4-methylpiperazin-1-yl)phenyl]amino}pyri midin-4-yl)piperazine-1-carboxamide;
*N*-(4-acetylphenyl)-4-[2-({4-[2-(pyrrolidin-1-yl)ethoxy]phenyl}amino)pyrim idin-4-yl]piperazine-1-carboxamide;
*N*-(4-acetylphenyl)-4-(2-{[4-(4-methylpiperidin-1-yl)phenyl]amino}pyrimidi n-4-yl)piperazine-1-carboxamide;
*N*-(4-acetylphenyl)-4-[2-({4-[2-(morpholin-4-yl)ethoxy]phenyl}amino)pyrim idin-4-yl]piperazine-1-carboxamide;

*N*-(4-acetylphenyl)-4-[2-({4-[3-(morpholin-4-yl)propoxy]phenyl}amino) pyrimidin-4-yl]piperazine-1-carboxamide;
*N*-(4-cyanophenyl)-4-(5-methyl-2-{[4-(morpholin-4-yl)phenyl]amino} pyrimidin-4-yl)piperazine-1-carboxamide;
*N*-(4-cyanophenyl)-4-(6-methyl-2-{[4-(morpholin-4-yl)phenyl]amino} pyrimidin-4-yl)piperazine-1-carboxamide;
*N*-(4-acetylphenyl)-4-(5-methyl-2-{[4-(morpholin-4-yl)phenyl]amino} pyrimidin-4-yl)piperazine-1-carboxamide;
*N*-(4-acetylphenyl)-4-(6-methyl-2-{[4-(morpholin-4-yl)phenyl]amino} pyrimidin-4-yl)piperazine-1-carboxamide;
*N*-(4-acetylphenyl)-4-(2-{[4-(morpholin-4-yl)phenyl]amino}thieno[3,2-*d*]pyrimidin-4-yl)piperazine-1-carboxamide;
N-(4-acetylphenyl)-4-(2-{[4-(morpholin-4-yl)phenyl]amino}quinazolin-4-yl)piperazine-1-carboxamide;
*N*-(4-acetylphenyl)-4-(5-trifluoromethyl-2-{[4-(morpholin-4-yl)phenyl] amino}pyrimidin-4-yl)piperazine-1-carboxamide;
*N*-(4-acetylphenyl)-4-(5-amino-2-{[4-(morpholin-4-yl)phenyl]amino} pyrimidin-4-yl)piperazine-1-carboxamide;
*N*-(4-acetylphenyl)-3-[(5-methyl-2-{[4-(morpholin-4-yl)phenyl]amino} pyrimidin-4-yl)amino]pyrrolidine-1-carboxamide;
*N*-(4-acetylphenyl)-4-[(5-methyl-2-{[4-(morpholin-4-yl)phenyl]amino} pyrimidin-4-yl)amino]piperidine-1-carboxamide; and
*N*-(4-acetylphenyl)-4-[5-methyl-2-({4-[3-(pyrrolidin-1-yl)propoxy]phenyl} amino)pyrimidin-4-yl]piperazine-1 -carboxamide.

According to some common methods in the art to which the present invention belongs, the compound of the general formula I of the present invention can form a pharmaceutically acceptable salt thereof with an acid. Preferred acids are hydrochloric acid, hydrobromic acid, hydrofluoric acid, sulfuric acid, phosphoric acid, nitric acid, formic acid, acetic acid, propionic acid, oxalic acid, malonic acid, succinic acid, fumaric acid, maleic acid, lactic acid, malic acid, tartaric acid, citric acid, picric acid, methanesulfonic acid, ethanesulfonic acid, toluenesulfonic acid, benzenesulfonic acid, naphthalenesulfonic acid, trifluoroacetic acid and aspartic acid.

Through the enzyme activity test of JAK2 and FLT3, it is found that the compound of the present invention has a significant inhibitory effect on the kinase activity of JAK2 and FLT3, and has a strong inhibitory effect on blood cancer cells with high JAK2 or FLT3 expression.

Through in vitro inhibition of human erythroleukemia cell HEL and human myeloid monocytic leukemia cell MV4-11 activity test, we found that the compound of the present invention has significant anti-tumor activity, so the compound of the present invention can be used in the manufacture of a medicament for treating or preventing various proliferative diseases or hematological malignancies, such as myelofibrosis, multiple myeloma, polycythemia vera, primary thrombocythemia, acute myeloid leukemia, acute lymphocytic leukemia, etc.

The exact amount of the compound of the present invention required for the treatment of proliferative diseases or hematological malignancies will vary from subject to subject, depending on the subject's type, age and general conditions, severity of the disease being treated, the specific compound used, and mode of administration, such as the route and frequency of administration, etc. A person of ordinary skill in the art can determine the appropriate effective amount only by using conventional experimental methods.

The dosage of the compound can start about 0.1 to 100 mg/kg body weight per day, preferably 1 to 50 mg/kg body weight/day. It is understood that the dosage may vary depending on the needs of the patient, the severity of the proliferative disease or hematological malignancy being treated, and the specific compound used. Moreover, it is understandable that the initial dose administered can be increased beyond the upper limit in order to quickly reach the required blood level, or the initial dose can be less than the optimal value, and the daily dose can be gradually increased during the treatment period, depending on the specific case. If necessary, the daily dose can also be divided into multiple doses, for example 2 to 4 times a day.

Mammals mean humans or animals.

The amount of the active ingredient (i.e. the compound according to the present invention) in the pharmaceutical composition and its unit dosage form can vary, depending on the specific application, the potency of the specific compound and the desired concentration. Generally speaking, the content of the active ingredient will be between 0.5% and 90%, based on the total weight of the composition.

In combination therapy, the compound of the present invention and the other compound may be administered simultaneously or at intervals. When administered simultaneously, the compound of the present invention and the other compound may be combined in a single pharmaceutical composition or in separate compositions.

The examples and preparation examples provided below further illustrate and exemplify the compounds of the present invention and the preparation methods thereof. It should be understood that the scope of the following examples and preparation examples does not limit the scope of the present invention in any way.

The following synthetic scheme describes the preparation of the derivatives of general formula I of the present invention. All raw materials are prepared by the methods described in these schematic diagrams, by methods well known to those of ordinary skill in the organic chemistry field, or are commercially available. All the final compounds of the present invention are prepared by the methods described in these schematic diagrams or by methods similar thereto,

which are well known to those of ordinary skill in the organic chemistry field. All the variable factors used in these diagrams are as defined below or as defined in the claims.

According to the compounds of general formula I of the present invention, in Scheme A, Scheme B, Scheme C and Scheme D, the following compounds are taken as examples: $R_1$ and $R_2$ are hydrogen or methyl. The definitions of the substituents $R_4$, $R_8$, m, and n are the same as in the claims.

Scheme A:

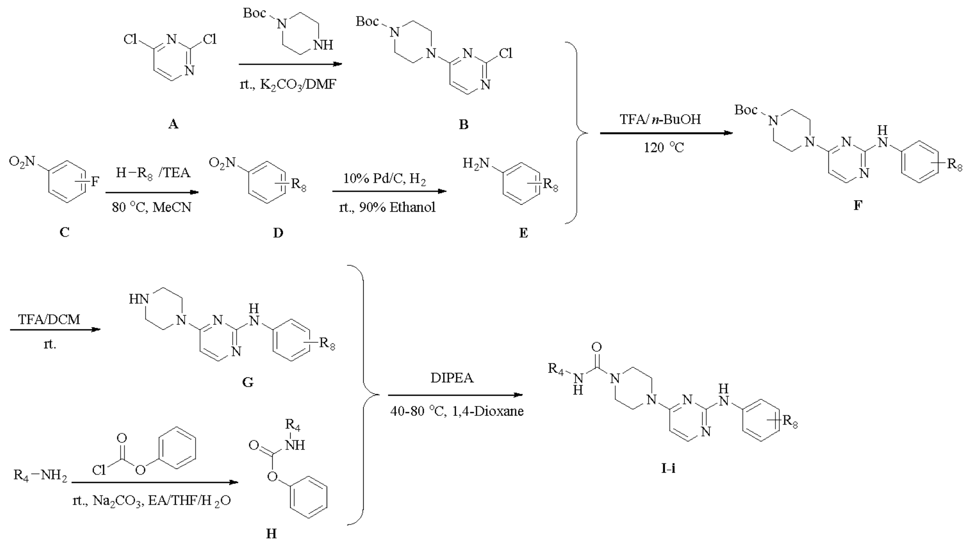


**Scheme A Synthesis of compound I-i**

In Scheme A, 2,4-dichloropyrimidine is used as the starting material and undergoes substitution reaction with potassium carbonate as the base and N-Boc piperazine to obtain intermediate **B,** which is then substituted with $R_8$ substituted aniline **E** to obtain intermediate **F** , followed by de-Boc protective group reaction with trifluoroacetic acid to obtain intermediate **G,** and followed by substitution reaction with $R_4$ substituted phenyl carbamate **H** to obtain compound I-i of general formula I.

Scheme B:

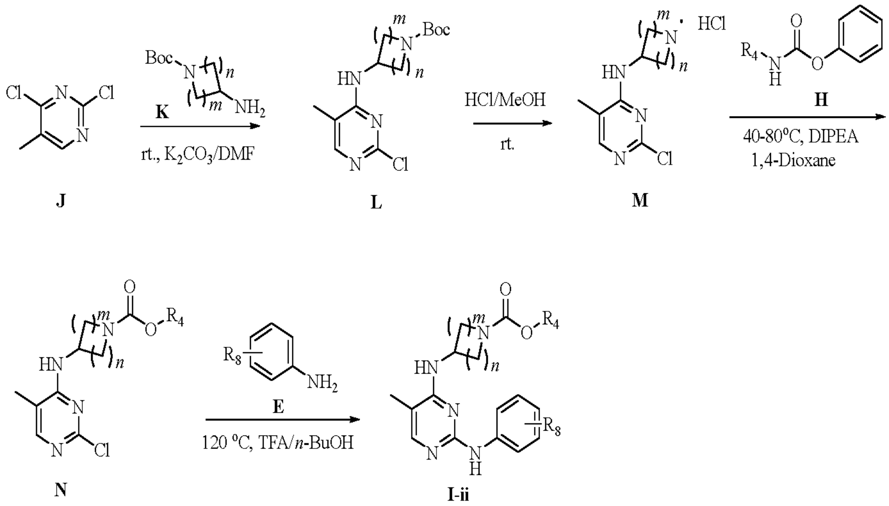


**Scheme B Synthesis of compound I-ii**

In Scheme B, 5-methyl-2,4-dichloropyrimidine **(J)** is used as the starting material and undergoes nucleophilic substitution reaction with the Boc-protected intermediate **K** using potassium carbonate as the base to obtain intermediate **L,** which is then subjected to de-Boc protection reaction in a hydrochloric acid in methanol solution to obtain intermediate **M,** followed by reaction with $R_4$ substituted phenyl carbamate **H** to obtain intermediate **N**, and followed by substitution reaction with $R_8$ substituted aniline **E** to obtain compound **I-ii** of general formula **I**.

Scheme C:

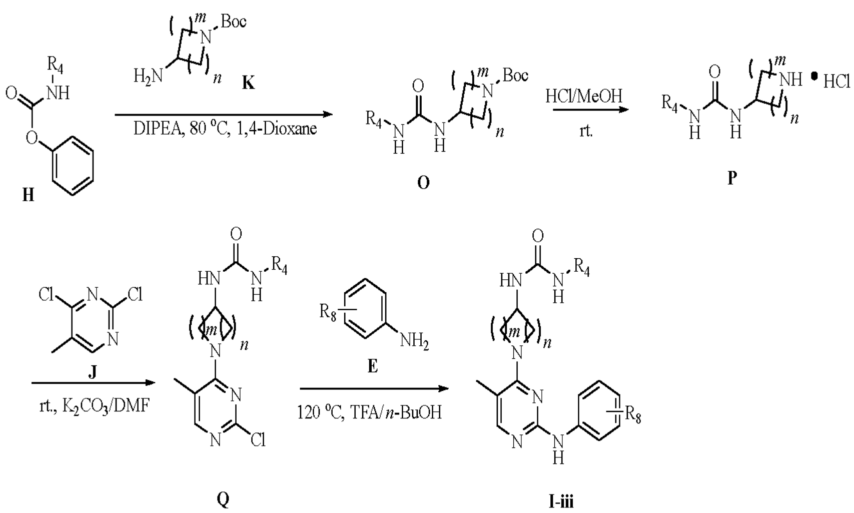


**Scheme C Synthesis of compound I-iii**

In Scheme C, the addition reaction of $R_4$-substituted phenyl carbamate **H** with Boc-protected intermediate **K** gives intermediate **O**, which is then de-Boc-protected in a hydrochloric acid in methanol solution to obtain intermediate **P,** followed by nucleophilic substitution reaction with 5-methyl -2,4-dichloropyrimidine (**J**) to obtain intermediate **Q,** and followed by substitution reaction with $R_8$ substituted aniline **E** to obtain compound **I-iii** of general formula I.

Scheme D:

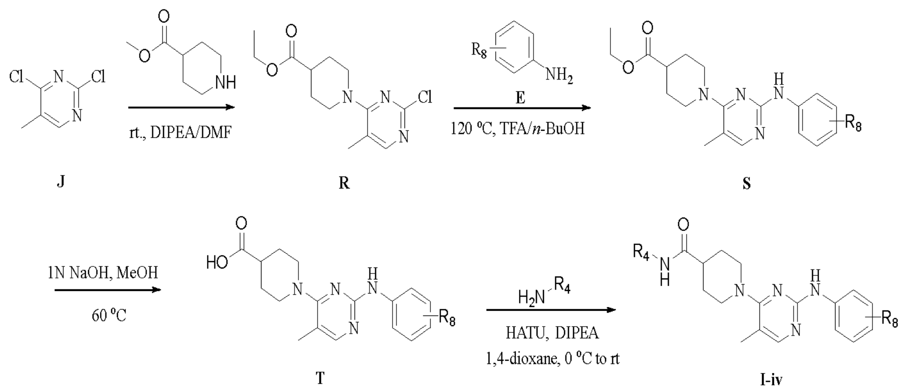


J R S T I-iv

**Scheme D Synthesis of compound I-iv**

In Scheme D, 5-methyl-2,4-dichloropyrimidine (**J**) is used as the starting material and undergoes nucleophilic substitution reaction with methyl 4-piperidinecarboxylate under the condition of DIPEA as the base to obtain intermediate **R**, which is then subjected to reaction with $R_8$ substituted aniline E to obtain intermediate **S,** followed by hydrolysis reaction in NaOH solution to obtain intermediate **T,** and followed by condensation reaction with $R_4$ substituted amine to obtain compound **I-iv** of general formula I.

## DETAILED DESCRIPTION OF THE INVENTION

In the following examples, methods for preparing some of the compounds are described. It should be understood that the following methods and other methods known to those of ordinary skill in the art can be applied to the preparation of all the compounds described in the present invention. The examples are intended to illustrate rather than limit the scope of the invention. The proton nuclear magnetic resonance spectrum of the compounds was measured with Bruker ARX-400 or Bruker ARX-600, and the mass spectrum was measured with Agilent 1100 LC/MSD; all reagents used were analytical or chemical pure.

### Examples

### Example 1 Preparation of *N*-(4-methoxyphenyl)-4-(2-{[4-(morpholin-4-yl)phenyl]amino}pyrimidin-4-yl)piperazine-1-carboxamide

#### 1.1. Synthesis of tert-butyl 4-(2-chloropyrimidin-4-yl)piperazine-1-carboxylate (B)

At room temperature, 20.0 g (134 mmol) of 2, 4-dichloropyrimidine (A) was dissolved in 200 mL of *N, N*-dimethylformamide (DMF), 22.0 g (161 mmol) of potassium carbonate was added, and 26.0 g (140 mmol) of *N*-BOC-piperazine was added in batches on ice bath. After that, the reaction was carried out at room temperature for 2 hours. After the reaction was completed, 1 L of water was added to the reaction solution and the reaction solution was stirred and filtered with suction, then the filter cake was washed with 100 mL of water, and dried to obtain a crude intermediate **E.** The crude intermediate **E** was purified by column chromatography to obtain 30.0 g of white solid with a yield of 75%.

#### 1.2. Synthesis of 4-(4-nitrophenyl)morpholine (D)

At room temperature, 20.0 g (142 mmol) of p-fluoronitrobenzene (**C**) was dissolved in 200 mL of acetonitrile, and 17.2 g (170 mmol) of triethylamine and 18.5 g (212 mmol) of morpholine were added to the reaction solution. After that, the temperature was raised to 80 °C and the reaction was carried out for 3 hours. After the reaction was completed, the reaction solution was cooled to room temperature, the solvent was concentrated under reduced pressure, and 200 mL of water was added to the residue, which was stirred at room temperature for 20 minutes and filtered with suction, then 100 mL of water was used to wash the filter cake, and 26.9 g of yellow solid was obtained after drying. The yield was 91%.

#### 1.3. Synthesis of 4-(4-aminophenyl)morpholine (E)

26.9 g (129 mmol) of intermediate D and 1.34 g (5% m/m) of 10% palladium on carbon were added into 500

mL of 90% ethanol solution, hydrogen was added, and the reaction was carried out at room temperature for 5 hours. After the reaction was completed, suction filtration was carried out, the filter cake was washed with 100 mL of 90% ethanol, and the filtrate was concentrated under reduced pressure to obtain 22.0 g of purple solid with a yield of 96%.

**1.4. Synthesis of tert-butyl 4-(2-{[4-(morpholin-4-yl)phenyl]amino} pyrimidin-4-yl)piperazine-1-carboxylate (F)**

5.0 g (16.8 mmol) of intermediate C and 3.6 g (20.2 mmol) of intermediate E was added to 50 mL of n-butanol, and 5.7 g (50.4 mmol) of trifluoroacetic acid was slowly added dropwise at room temperature. After the addition, the temperature was raised to 120°C and the reaction was carried out at 120°Cfor 2 hours. After the reaction was completed, the solvent was concentrated under reduced pressure, 50 mL of water was added to the residue, the pH was adjusted to 8 with saturated sodium bicarbonate solution, and suction filtration was carried out. The filter cake was washed with water (20 mL) and dried to obtain 6.9 g of a blue-white solid with a yield of 93%.

**1.5. Synthesis of 4-(2-{[4-(morpholin-4-yl)phenyl]amino}pyrimidin-4-yl)piperazine (G)**

At room temperature, 6.9 g (15.6 mmol) of intermediate G was added to 70 mL of dichloromethane, 70 mL of trifluoroacetic acid was slowly added dropwise, and the reaction was carried out at room temperature for 4 hours. After the reaction was completed, the solution was concentrated under reduced pressure, 50 mL of water was added to the residue, the pH was adjusted to 8 with saturated sodium bicarbonate solution, and suction filtration was carried out. The filter cake was washed with water (20 mL) and dried to obtain a gray solid 4.1 g with a yield of 77%.

**1.6. Synthesis of *N*-(4-methoxyphenyl) phenyl carbamate (H)**

At room temperature, 1.4 g (11.2 mmol) 4-methoxyaniline and 0.7 g (6.7 mmol) sodium carbonate were added to 10 mL of ethyl acetate-tetrahydrofuran-water (3:1:1) mixed solution, a solution of 1.9 g (12.3 mmol) of phenyl chloroformate in ethyl acetate (3 mL) was added dropwise under ice bath. After the addition, the reaction was carried out at room temperature for 2 hours. After the reaction was completed, the reaction solution was concentrated under reduced pressure, 10 mL of water was added to the residue, the pH was adjusted to 5 with 1M hydrochloric acid solution, and suction filtration was carried out. The filter cake was washed with water (10 mL), and after drying, 2.5 g of a white solid was obtained. The yield was 91%.

**1.7. Synthesis of *N*-(4-methoxyphenyt)-4-(2-{[4-(morpholin-4-yl)phenyl] amino}pyrimidin-4-yl)piperazine-1-carboxamide(I)**

At room temperature, 0.20 g (0.6 mmol) of intermediate **G** and 0.17 g (0.7 mmol) of intermediate **H** were added to 4 mL of 1,4-dioxane, and 0.30 g (2.3 mmol) of *N, N*-diisopropylethylamine (DIPEA) was added dropwise to the reaction solution. After the addition, the temperature was raised to 80°C and the reaction was carried out at 80°C for 3 hours. After the reaction is completed, the reaction solution was cooled to room temperature, and the solvent was concentrated under reduced pressure. 10 mL of water and 2.5% NaOH solution was added to the residue to adjust the pH to 10, and suction filtration and drying were carried out to obtain a crude product. The crude product was purified by column chromatography (dichloromethane:methanol=20:1) to obtain 0.15 g of white solid with a yield of 62%.

**Example 2. Preparation of *N*-(4-trifluoromethoxyphenyl)-4-(2-{[4-( morpholin-4-yl)phenyl]amino}pyrimidin-4-yl)piperazine-1-carboxamide**

Using 4-trifluoromethoxyaniline as a raw material, the key intermediate **H** was synthesized according to the synthesis method of item 1.6 in Example 1, and then *N*-(4-trifluoromethoxyphenyl)-4-(2-{[4-(morpholin-4-yl)phenyl]amino} pyrimidin-4-yl)piperazine-1-carboxamide was synthesized according to the synthesis method of item 1.7 in Example 1. The yield was 65%.

**Example 3. Preparation of *N*-(4-ethylphenyl)-4-(2-{[4-(morpholin-4-yl)phenyl]amino}pyrimidin-4-yl)piperazine-1-carboxamide**

Using 4-ethylaniline as a raw material, the key intermediate **H** was synthesized according to the synthesis method of item 1.6 in Example 1, and then *N*-(4-ethylphenyl)-4-(2-{[4-(morpholin-4-yl)phenyl]amino}pyrimidin-4 -yl)piperazine-1-carboxamide was synthesized according to the synthesis method of item 1.7 in Example 1. The yield was 57%.

**Example 4. Preparation of *N*-phenyl-4-(2-{[4-(morpholin-4-yl)phenyl] amino}pyrimidin-4-yl)piperazine-1-carboxamide**

Using aniline as a raw material, the key intermediate **H** was synthesized according to the synthesis method of item 1.6 in Example 1, and then *N*-phenyl-4-(2-{[4-(morpholin-4-yl)phenyl]amino}pyrimidin-4-yl) piperazine-1-carboxamide was synthesized according to the synthesis method of item 1.7 in Example 1. The yield was 64%.

**Example 5. Preparation of *N*-(4-methoxyformylphenyl)-4-(2-{[4-( morpholin-4-yl)phenyl] amino}pyrimidin-4-yl)piperazine-1-carboxamide**

Using methyl 4-aminobenzoate as a raw material, the key intermediate **H** was synthesized according to the synthesis method of item 1.6 in Example 1, and then *N*-(4-methoxyformylphenyl)-4-(2-{[4-(morpholin-4-yl)phenyl]amino} pyrimidin-4-yl)piperazine-1-carboxamide was synthesized according to the synthesis method of item 1.7 in Example 1. The yield was 64%.

**Example 6. Preparation of *N*-(4-acetytphenyt)-4-(2-{[4-(morpholin-4-yl) phenyl] amino}pyrimidin-4-yl)piperazine-1-carboxamide**

Using 4-aminoacetophenone as a raw material, the key intermediate **H** was synthesized according to the synthesis method of item 1.6 in Example 1, and then *N*-(4-acetylphenyl)-4-(2-{[4-(morpholin-4-yl)phenyl]amino}pyrimidin-4-yl)piperazine-1-carboxamide was synthesized according to the synthesis method of item 1.7 in Example 1. The yield was 62%.

**Example 7. Preparation of *N*-(4-nitrophenyt)-4-(2-{[4-(morpholin-4-yl)phenyl]amino}pyrimidin-4-yl)piperazine-1-carboxamide**

Using 4-nitroaniline as a raw material, the key intermediate **H** was synthesized according to the synthesis method of item 1.6 in Example 1, and then *N*-(4-nitrophenyl)-4-(2-{[4-(morpholin-4-yl)phenyl]amino}pyrimidin-4-yl)piperazine-1-carboxamide was synthesized according to the synthesis method of item 1.7 in Example 1. The yield was 45%.

**Example 8. Preparation of *N*-(4-cyano-3-fluorophenyl)-4-(2-{[4-( morpholin-4-yl)phenyl] amino}pyrimidin-4-yl)piperazine-1-carboxamide**

Using 4-cyano-3-fluoroaniline as a raw material, the key intermediate **H** was synthesized according to the synthesis method of item 1.6 in Example 1, and then *N*-(4-cyano-3-fluorophenyl)-4-(2-{[4-(morpholin-4-yl)phenyl]amino} pyrimidin-4-yl)piperazine-1-carboxamide was synthesized according to the synthesis method of item 1.7 in Example 1. The yield was 71%.

**Example 9. Preparation of *N*-(4-isopropoxyphenyl)-4-(2-{[4-(morpholin-4-yl)phenyl]amino}pyrimidin-4-yl)piperazine-1-carboxamide**

Using 4-isopropoxyaniline as a raw material, the key intermediate **H** was synthesized according to the synthesis method of item 1.6 in Example 1, and then *N*-(4-isopropoxyphenyl)-4-(2-{[4-(morpholin-4-yl)phenyl]amino}pyrimidin-4-yl)piperazine-1-carboxamide was synthesized according to the synthesis method of item 1.7 in Example 1. The yield was 70%.

**Example 10. Preparation of *N*-(4-carbamoylphenyl)-4-(2-{[4-(morpholin-4-yl)phenyl]amino}pyrimidin-4-yl)piperazine-1-carboxamide**

Using 4-aminobenzamide as a raw material, the key intermediate **H** was synthesized according to the synthesis method of item 1.6 in Example 1, and then *N*-(4-carbamoylphenyl)-4-(2-{[4-(morpholin-4-yl)phenyl]amino}pyrimidin-4-yl)piperazine-1-carboxamide was synthesized according to the synthesis method of item 1.7 in Example 1. The yield was 52%.

**Example 11. Preparation of *N*-(4-methylcarbamoylphenyl)-4-(2-{[4-( morpholin-4-yl)phenyl]amino}pyrimidin-4-yl)piperazine-1-carboxamide**

Using 4-amino-*N*-methylbenzamide as a raw material, the key intermediate **H** was synthesized according to

the synthesis method of item 1.6 in Example 1, and then *N*-(4-methylcarbamoylphenyl)-4-(2-{[4-(morpholin-4-yl)phenyl] amino}pyrimidin-4-yl)piperazine-1-carboxamide was synthesized according to the synthesis method of item 1.7 in Example 1. The yield was 61%.

**Example 12. Preparation of *N*-(4-aminosulfonylphenyl)-4-(2-{[4-( morpholin-4-yl)phenyl] amino}pyrimidin-4-yl)piperazine-1-carboxamide**

Using 4-aminobenzenesulfonamide as a raw material, the key intermediate **H** was synthesized according to the synthesis method of item 1.6 in Example 1, and then *N*-(4-aminosulfonylphenyl)-4-(2-{[4-(morpholin-4-yl)phenyl] amino}pyrimidin-4-yl)piperazine-1-carboxamide was synthesized according to the synthesis method of item 1.7 in Example 1. The yield was 47%.

**Example 13. Preparation of *N*-(4-acetylaminophenyl)-4-(2-{[4-( morpholin-4-yl)phenyl] amino}pyrimidin-4-yl)piperazine-1-carboxamide**

Using 4-aminoacetanilide as a raw material, the key intermediate **H** was synthesized according to the synthesis method of item 1.6 in Example 1, and then *N*-(4-acetylaminophenyl)-4-(2-{[4-(morpholin-4-yl)phenyl]amino}pyrimidin-4-yl)piperazine-1-carboxamide was synthesized according to the synthesis method of item 1.7 in Example 1. The yield was 53%.

**Example 14. Preparation of *N*-(4-methanesulfonylphenyl)-4-(2-{[4-( morpholin-4-yl)phenyl] amino}pyrimidin-4-yl)piperazine-1-carboxamide**

Using 4-methanesulfonylaniline as a raw material, the key intermediate **H** was synthesized according to the synthesis method of item 1.6 in Example 1, and then *N*-(4-methanesulfonylphenyl)-4-(2-{[4-(morpholin-4-yl)phenyl]amino}pyrimidin-4-yl)piperazine-1-carboxamide was synthesized according to the synthesis method of item 1.7 in Example 1. The yield was 68%.

**Example 15. Preparation of *N*-(4-carbamoylphenyl)-4-(2-{[4-(4-methylpiperazin-1-yl)phenyl]amino}pyrimidin-4-yl)piperazine-1-carbox amide**

Using *N*-methylpiperazine as a raw material, the key intermediate **G** was synthesized according to the synthesis method of items 1.1 to 1.5 in Example 1; using 4-aminobenzamide as a raw material, the key intermediate **H** was synthesized according to the synthesis method of item 1.6 in Example 1, then *N*-(4-carbamoylphenyl)-4-(2-{[4-(4-methylpiperazin-1-yl)phenyl]amino}pyrimidin-4-yl)piperazine-1-carboxamide was synthesized according to the synthesis method of item 1.7 in Example 1. The yield was 56%.

**Example 16. Preparation of *N*-(4-acetylphenyl)-4-[2-({4-[2-(pyrrolidin-1-yl)ethoxy]phenyl}amino)pyrimidin-4-yl] piperazine-1-carboxamide**

Using 1-(2-hydroxyethyl)pyrrolidine as a raw material, the key intermediate **G** was synthesized according to the synthesis method of items 1.1 to 1.5 in Example 1; using 4-aminoacetophenone as a raw material, the key intermediate **H** was synthesized according to the synthesis method of item 1.6 in Example 1, then *N*-(4-acetylphenyl)-4-[2-({4-[2-(pyrrolidin-1-yl)ethoxy]phenyl}amino)pyrimidin-4-yl]piperazine-1-carboxamide was synthesized according to the synthesis method of item 1.7 in Example 1. The yield was 42%.

**Example 17. Preparation of *N*-(4-acetylphenyl)-4-(2-{[4-(4-methylpiperidin-1-yl)phenyl]amino)pyrimidin-4-yl)piperazine-1-carboxamide;**

Using 4-methylpiperidine as a raw material, the key intermediate **G** was synthesized according to the synthesis method of items 1.1 to 1.5 in Example 1; using 4-aminoacetophenone as a raw material, the key intermediate **H** was synthesized according to the synthesis method of item 1.6 in Example 1, then *N*-(4-acetylphenyl)-4-(2-{[4-(4-methylpiperidin-1-yl)phenyl]amino}pyrimidin-4-yl)piperazine-1-carboxamide was synthesized according to the synthesis method of item 1.7 in Example 1. The yield was 51%.

**Example 18. Preparation of *N*-(4-acetylphenyl)-4-[2-({4-[2-(morpholin-4 -yl)ethoxy]phenyl}amino)pyrimidin-4-yl] piperazine-1-carboxamide**

Using 4-(2-hydroxyethyl)morpholine as a raw material, the key intermediate **G** was synthesized according to the synthesis method of items 1.1 to 1.5 in Example 1; using 4-aminoacetophenone as a raw material, the key intermediate **H** was synthesized according to the synthesis method of item 1.6 in Example 1, then *N*-(4-acetylphenyl)-4-[2-({4-[2-(morpholin-4-yl)ethoxy]phenyl}amino)pyrimidin-4-yl]piperazine-1-carboxamide was synthesized according to the synthesis method of item 1.7 in Example 1. The yield was 52%.

**Example 19. Preparation of *N*-(4-acetylphenyl)-4-[2-({4-[3-(morpholin-4 -yl)propoxy]phenyl}amino)pyrimidin-4-yl]piperazine-1-carboxamide**

Using 4-(3-hydroxypropyl) morpholine as a raw material, the key intermediate **G** was synthesized according to the synthesis method of items 1.1 to 1.5 in Example 1; using 4-aminoacetophenone as a raw material, the key intermediate **H** was synthesized according to the synthesis method of item 1.6 in Example 1, then *N*-(4-acetylphenyl)-4-[2-({4-[3-(morpholin-4-yl)propoxy]phenyl} amino)pyrimidin-4-yl]piperazine-1-carboxamide was synthesized according to the synthesis method of item 1.7 in Example 1. The yield was 41%.

**Example 20. Preparation of *N*-(4-cyanophenyl)-4-(5-methyl-2-{[4-( morpholin-4-yl)phenyl] amino}pyrimidin-4-yl)piperazine-1-carboxamide**

Using 5-methyl-2,4-dichloropyrimidine as a raw material, the key intermediate **G** was synthesized according to the synthesis method of items 1.3 to 1.5 in Example 1; using 4-cyanoaniline as a raw material, the key intermediate **H** was synthesized according to the synthesis method of item 1.6 in Example 1, then *N*-(4-cyanophenyl)-4-(5-methyl-2-{[4-(morpholin-4-yl)phenyl]amino}pyrimidin-4-yl)piperazine-1-carboxamide was synthesized according to the synthesis method of item 1.7 in Example 1. The yield was 70%.

**Example 21. Preparation of *N*-(4-cyanophenyl)-4-(6-methyl-2-{[4-( morpholin-4-yl)phenyl] amino}pyrimidin-4-yl)piperazine-1-carboxamide**

Using 6-methyl-2,4-dichloropyrimidine as a raw material, the key intermediate **G** was synthesized according to the synthesis method of items 1.3 to 1.5 in Example 1; using 4-cyanoaniline as a raw material, the key intermediate **H** was synthesized according to the synthesis method of item 1.6 in Example 1, then *N*-(4-cyanophenyl)-4-(6-methyl-2-{[4-(morpholin-4-yl)phenyl]amino}pyrimidin-4-yl)piperazine-1-carboxamide was synthesized according to the synthesis method of item 1.7 in Example 1. The yield was 51%.

**Example 22. Preparation of *N*-(4-acetylphenyl)-4-(5-methyl-2-{[4-( morpholin-4-yl)phenyl] amino}pyrimidin-4-yl)piperazine-1-carboxamide**

Using 5-methyl-2,4-dichloropyrimidine as a raw material, the key intermediate **G** was synthesized according to the synthesis method of items 1.3 to 1.5 in Example 1; using 4-aminoacetophenone as a raw material, the key intermediate H was synthesized according to the synthesis method of item 1.6 in Example 1, then *N*-(4-acetylphenyl)-4-(5-methyl-2-{[4-(morpholin-4-yl)phenyl]amino}pyrimidin-4-yl)piperazine-1-carboxamide was synthesized according to the synthesis method of item 1.7 in Example 1. The yield was 64%.

**Example 23. Preparation of *N*-(4-acetylphenyl)-4-(6-methyl-2-{[4-( morpholin-4-yl)phenyl] amino}pyrimidin-4-yl)piperazine-1-carboxamide**

Using 6-methyl-2,4-dichloropyrimidine as a raw material, the key intermediate **G** was synthesized according to the synthesis method of items 1.3 to 1.5 in Example 1; using 4-aminoacetophenone as a raw material, the key intermediate **H** was synthesized according to the synthesis method of item 1.6 in Example 1, then *N*-(4-acetylphenyl)-4-(6-methyl-2-{[4-(morpholin-4-yl)phenyl]amino}pyrimidin-4-yl)piperazine-1-carboxamide was synthesized according to the synthesis method of item 1.7 in Example 1. The yield was 62%.

**Example 24. Preparation of *N*-(4-acetylphenyl)-4-(2-{[4-(morpholin-4-yl)phenyl]amino}thieno[3,2-*d*]pyrimidin-4-yl)piperazine-1-carboxamide**

Using 2,4-dichlorothieno[3,2-d]pyrimidine as a raw material, the key intermediate **G** was synthesized according

to the synthesis method of items 1.3 to 1.5 in Example 1; using 4-aminoacetophenone as a raw material, the key intermediate **H** was synthesized according to the synthesis method of item 1.6 in Example 1, then *N*-(4-acetylphenyl)-4-(2-{[4-(morpholin-4-yl)phenyl]amino}thieno[3,2-*d*]pyrimidin-4-yl)piperazine-1-carboxamide was synthesized according to the synthesis method of item 1.7 in Example 1. The yield was 60%.

**Example 25. Preparation of *N*-(4-acetylphenyl)-4-(2-{[4-(morpholin-4-yl)phenyl]amino}quinazolin-4-yl)piperazine-1-carboxamide**

Using 2,4-dichloroquinazoline as a raw material, the key intermediate **G** was synthesized according to the synthesis method of items 1.3 to 1.5 in Example 1; using 4-aminoacetophenone as a raw material, the key intermediate **H** was synthesized according to the synthesis method of item 1.6 in Example 1, then *N*-(4-acetylphenyl)-4-(2-{[4-(morpholin-4-yl)phenyl]amino}quinazolin-4-yl)piperazine-1-carboxamide was synthesized according to the synthesis method of item 1.7 in Example 1. The yield was 65%.

**Example 26. Preparation of *N*-(4-acetylphenyl)-4-(5-trifluoromethyl-2-{[ 4-(morpholin-4-yl)phenyl]amino}pyrimidin-4-yl)piperazine-1-carboxamide**

Using 5-trifluoromethyl-2,4-dichloropyrimidine as a raw material, the key intermediate **G** was synthesized according to the synthesis method of items 1.3 to 1.5 in Example 1; using 4-aminoacetophenone as a raw material, the key intermediate **H** was synthesized according to the synthesis method of item 1.6 in Example 1, then *N*-(4-acetylphenyl)-4-(5-trifluoromethyl-2-{[4-(morpholin-4-yl)phenyl]amino}pyrimidin-4-yl)piperazine-1-carboxamide was synthesized according to the synthesis method of item 1.7 in Example 1. The yield was 61%.

**Example 27. Preparation of *N*-(4-acetylphenyl)-4-(5-amino-2-{[4-( morpholin-4-yl)phenyl] amino}pyrimidin-4-yl)piperazine-1-carboxamide**

Using 5-nitro-2,4-dichloropyrimidine as a raw material, the key intermediate **G** was synthesized according to the synthesis method of items 1.3 to 1.5 in Example 1; using 4-aminoacetophenone as a raw material, the key intermediate **H** was synthesized according to the synthesis method of item 1.6 in Example 1, then *N*-(4-acetylphenyl)-4-(5-amino-2-{[4-(morpholin-4-yl)phenyl]amino}pyrimidin-4-yl)piperazine-1-carboxamide was synthesized according to the synthesis method of item 1.2 in Example 1. The yield was 30%.

**Example 28. Preparation of *N*-(4-acetylphenyl)-3-[(5-methyl-2-{[4-(morpholin-4-yl)phenyl[amino}pyrimidin-4-yl)amino[pyrrolidine-1-carboxamide**

**28.1. Synthesis of tert-butyl 3-[(2-chloro-5-methylpyrimidin-4-yl)amino]pyrrolidine-1-carboxylate (L)**

At room temperature, 20.0 g (125 mmol) of 5-methyl-2,4-dichloropyrimidine (**J**) was dissolved in 200 mL of *N,N*-dimethylformamide (DMF), 20.5 g (150 mmol) of potassium carbonate was added, and 26.0 g (138 mmol) of tert-butyl 3-aminopyrrolidine-1-carboxylate (**K**) was added in batches on ice bath. After that, a reaction was carried out at room temperature for 2 hours. After the reaction was completed, 1 L of water was added to the reaction solution, and the reaction solution was stirred and filtered with suction, then the filter cake was washed with 100 mL of water, and dried to obtain a crude intermediate **L.** The crude intermediate **L** was purified by column chromatography to obtain 29.3 g of white solid with a yield of 75%.

**28.2. Synthesis of 3-[(2-chloro-5-methylpyrimidin-4-yl)amino]pyrrolidine hydrochloride (M)**

At room temperature, 7 g (22.4 mmol) of intermediate **L** was added to 70 mL of hydrochloric acid in methanol solution, and reacted at room temperature for 12 hours. After the reaction was completed, the solution was concentrated under reduced pressure and filtered with suction. The filter cake was washed with ethanol (10 mL) and dried to obtain 4.1 g of a gray solid with a yield of 86%.

**28.3 Synthesis of *N*-(4-acetylphenyl)-3-[(2-chloro-5-methylpyrimidin-4-yl)amino]pyrrolidine-1-carboxamide (N)**

Using 4-aminoacetophenone as a raw material, the key intermediate **H** was synthesized according to the synthesis method of item 1.6 in Example 1, and using 3-[(2-chloro-5-methylpyrimidin-4-yl)amino]pyrrolidine hydrochloride (M) as a raw material, the key intermediate *N*-(4-acetylphenyl)-3-[(2-chloro-5-methylpyrimidin-4-yl)amino]pyrrolidine-1-carboxamide was synthesized according to the synthesis method of item 1.7 in Example 1. The yield was 60%.

**28.4 Synthesis of *N*-(4-acetylphenyl)-3-[(5-methyl-2-{[4-(morpholin-4-yl)phenyl]amino)pyrimidin-4-yl)amino]pyrrolidine-1-carboxamide**

Using *N*-(4-acetylphenyl)-3-[(2-chloro-5-methylpyrimidin-4-yl)amino]pyrrolidine-1-carboxamide (**N**) as a raw material, *N*-(4-acetylphenyl)-3-[(5-methyl-2-{[4-(morpholin-4-yl)phenyl]amino}pyrimidin-4-yl)amino]pyrrolidine-1-carboxamide was synthesized according to the synthesis method of item 1.4 in Example 1. The yield was 81%.

**Example 29. Preparation of 1-(4-acetylphenyl)-3-[1-(5-methyl-2-{[4-(morpholin-4-yl)phenyl]amino}pyrimidin-4-yl)pyrrolidin-3-yl]urea**

Using 4-aminoacetophenone as a raw material, the key intermediate **H** was synthesized according to the synthesis method of item 1.6 in Example 1. At room temperature, 5.0 g (26.8 mmol) of tert-butyl 3-aminopyrrolidine-1-carboxylate (**K**) and 7.5 g (29.5 mmol) of intermediate **H** were added to 50 mL of 1,4-dioxane, and 140. g (107.2 mmol) of *N, N*-diisopropylethylamine (DIPEA) was added dropwise to the reaction solution. After the addition, the temperature was raised to 80°C and the reaction was carried out at 80°C for 3 hours. After the reaction is completed, the reaction solution was cooled to room temperature, and the solvent was concentrated under reduced pressure. 10 mL of water and 2.5% NaOH solution was added to the residue to adjust the pH to 10, and suction filtration and drying were carried out to obtain a crude product. The crude product was purified by column chromatography (dichloromethane:methanol=20:1) to obtain 5.8 g of white solid with a yield of 62%.

**29.2. Synthesis of 1-(4-acetylphenyl)-3-(pyrrolidin-3-yl)urea hydrochloride (P)**

Using intermediate **O** as a raw material, 1-(4-acetylphenyl)-3-(pyrrolidin-3-yl)urea hydrochloride was synthesized according to the synthesis method of item 28.2 in Example 28. The yield was 89%.

**29.3. Synthesis of 1-(4-acetylphenyl)-3-[1-(2-chloro-5-methylpyrimidin-4 -yl)pyrrolidin-3-yl]urea (Q**)

Using intermediate **P** and 5-methyl-2,4-dichloropyrimidine as raw materials, 1-(4-acetylphenyl)-3-[1-(2-chloro-5-methylpyrimidin-4-yl)pyrrolidin-3-yl]urea was synthesized according to the synthesis method of item 1.3 in Example 1. The yield was 74%.

**29.4. Synthesis of 1-(4-acetylphenyl)-3-[1-(5-methyl-2-{[4-(morpholin-4-yl)phenyl]amino}pyrimidin-4-yl)pyrrolidine-3-yl]urea**

Using intermediate **Q** as a raw material, 1-(4-acetylphenyl)-3-[1-(5-methyl-2-{[4-(morpholin-4-yl)phenyl]amino} pyrimidin-4-yl)pyrrolidine- 3-yl]urea was synthesized according to the synthesis method of item 1.4 in Example 1. The yield was 65%.

**Example 30. Preparation of *N*-(4-acetylphenyl)-3-[(5-methyl-2-{[4-(morpholin-4-yl)phenyl]amino}pyrimidin-4-yl)amino]azetidine-1-carboxamide**

Using tert-butyl 3-aminoazetidine-1-carboxylate as a raw material, N-(4-acetylphenyl)-3-[(5- methyl-2-{[4-(morpholin-4-yl)phenyl]amino}pyrimidin-4-yl)amino]azetidine-1-carboxamide was synthesized according to the synthesis method of items 28.1 to 28.4 in Example 28. The yield was 68%.

**Example 31. Preparation of 1-(4-acetylphenyl)-3-[1-(5-methyl-2-{[4-(morpholin-4-yl)phenyl]amino}pyrimidin-4-yl) azetidin -3-yl]urea**

Using tert-butyl 3-aminoazetidine-1-carboxylate as raw material, 1-(4-acetylphenyl)-3-[1-( 5-methyl-2-{[4-(morpholin-4-yl)phenyl]amino}pyrimidin-4-yl)azetidin-3-yl]urea was synthesized according to the synthesis method of items 29.1 to 29.4 in Example 29. The yield was 61%.

**Example 32. Preparation of *N*-(4-acetylphenyl)-4-[(5-methyl-2-{[4-(morpholin-4-yl)phenyl]amino}pyrimidin-4-yl)amino]piperidine-1-carboxamide**

Using tert-butyl 4-amperidine-1-carboxylate as a raw material, *N*-(4-acetylphenyl)-4-[(5-methyl-2-{[4-(morpholin-4-yl)phenyl]amino}pyrimidin-4-yl)amino]piperidine-1-carboxamide was synthesized according to the synthesis method of items 28.1 to 28.4 in Example 28. The yield was 71%.

**Example 33. Preparation of *N*-(4-acetylphenyl)-4-[5-methyl-2-{[4-(morpholin-4-yl)phenyl]amino}pyrimidin-4-yl] piperazine-1-carboxamide**

**33.1 Synthesis of ethyl 1-(2-chloro-5-methylpyrimidin-4-yl)piperidine-4-carboxylate (R)**

Using 2,4-dichloro-5-methylpyrimidine (**J**) and ethyl piperidine-4-carboxylate as raw materials, ethyl 1-(2-chloro-5-methylpyrimidin-4-yl)piperidine-4-carboxylate was synthesized according to the synthesis method of item 1.3 in Example 1. The yield was 90%.

**33.2 Synthesis of ethyl 1-(5-methyl-2-{[4-(morpholin-4-yl)phenyl]amino}pyrimidin-4-yl)piperidine-4-carboxylate (S)**

Using ethyl 1-(2-chloro-5-methylpyrimidin-4-yl)piperidine-4-carboxylate (R) as a raw material, ethyl 1-(5-methyl-2-{[4-(morpholin-4-yl)phenyl]amino}pyrimidin-4-yl)piperidine-4-carboxylate was synthesized according to the synthesis method of item 1.4 in Example 1. The yield was 92%.

**33.3 Synthesis of 1-(5-methyl-2-{[4-(morpholin-4-yl)phenyl]amino} pyrimidine-4-yl) piperidine-4-carboxylic acid**

At room temperature, 5.0 g (11.8 mmol) of intermediate S was added to 50 mL of methanol, and 10 mL (1 mol/L) of sodium hydroxide solution was slowly added dropwise to the reaction solution. After the addition, the temperature was raised to 60°Cand the reaction was carried out at 60°Cfor 2 hours. After the reaction was completed, the reaction solution was cooled to room temperature. The reaction solution was then concentrated under reduced pressure. 50 mL of water was added to the residue, the pH was adjusted to 3 with 10% hydrochloric acid, and a large amount of solids precipitated out. The suction filtration was carried out and the filter cake was washed with water and dried under vacuum to obtain 0.63 g of a light yellow solid with a yield of 75%.

**33.4. Synthesis of *N*-(4-acetylphenyl)-1-(5-methyl-2-{[4-(morpholin-4-yl)phenyl]amino}pyrimidin-4-yl)piperidine-4-carboxamide**

At room temperature, 2.0 g (5.0 mmol) of intermediate **T** was dissolved in 100 mL of 1,4-dioxane, and 0.9 g (7.5 mmol) of *N,N*-diisopropylethylamine, 2.8 g (7.5 mmol) of HATU and 0.8 g (6.0 mmol) of 4-aminoacetophenone were added to the reaction solution. After the addition, the reaction was carried out at room temperature for 8 hours. After the reaction was completed, the reaction solution was cooled to room temperature, the reaction solution was concentrated under reduced pressure, and the crude product was purified by column chromatography to obtain a light yellow solid of *N*-(4-acetylphenyl)-1-(5-methyl-2-{[4-(morpholin-4-yl)phenyl]amino}pyrimidin-4-yl)piperidine -4-carboxamide with a yield of 55%.

**Example 34. Preparation of N-(4-acetylphenyl)-4-[5-methyl-2-({4-[3-(pyrrolidin-1-yl)propoxy]phenyl}amino)pyrimidin-4-yl]piperazine-1-carboxamide**

Using 1-(3-hydroxypropyl)pyrrolidine as a raw material, the key intermediate **E** was synthesized according to the synthesis method of items 1.1 to 1.2 in Example 1; using 5-methyl-2,4-dichloropyrimidine as a raw material, the key intermediate **G** was synthesized according to the synthesis method of items 1.3 to 1.5 in Example 1; using 4-aminoacetophenone as a raw material, the key intermediate **H** was synthesized according to the synthesis method of item 1.6 in Example 1, then *N*-(4-acetylphenyl)-4-[5-methyl-2-({4-[3-(pyrrolidin-1-yl)propoxy]phenyl}amino)pyrimidin-4-yl]piperazine-1-carboxamide was synthesized according to the synthesis method of item 1.7 in Example 1. The yield was 45%.

**Example 35. Preparation of *N*-(4-acetylphenyl)-4-(5-methyl-2-{[3-fluoro-4-(morpholin-4-yl)phenyl]amino}pyrimidin-4-yl)piperazine-1-carboxamide**

Using 2,4-difluoronitrobenzene as a raw material, the key intermediate **E** was synthesized according to the synthesis method of items 1.1 to 1.2 in Example 1; using 5-methyl-2,4-dichloropyrimidine as a raw material, the key intermediate **G** was synthesized according to the synthesis method of items 1.3 to 1.5 in Example 1; using 4-aminoacetophenone as a raw material, the key intermediate **H** was synthesized according to the synthesis method of item 1.6 in Example 1, then *N*-(4-acetylphenyl)-4-(5-methyl-2-{[3-fluoro-4-(morpholin-4-yl)phenyl]amino}pyrimidin-4-yl)piperazine-1-carboxamide was synthesized according to the synthesis method of item 1.7 in Example 1. The yield was 61%.

**Example 36. Preparation of *N*-(4-acetylphenyl)-4-(5-methyl-2-{[3-acetylamino-4-(morpholin-4-yl)phenyl] amino} pyrimidin-4-yl) piperazine-1-carboxamide**

Using 2-acetylamino-4-fluoronitrobenzene as a raw material, the key intermediate **E** was synthesized according to the synthesis method of items 1.1 to 1.2 in Example 1; using 5-methyl-2,4-dichloropyrimidine as a raw material, the key intermediate **G** was synthesized according to the synthesis method of items 1.3 to 1.5 in Example 1; using 4-aminoacetophenone as a raw material, the key intermediate **H** was synthesized according to the synthesis method of item 1.6 in Example 1, then *N*-(4-acetylphenyl)-4-(5-methyl-2-{[3-acetylamino-4-(morpholin-4-yl)phenyl]amino}pyrimidin-4-yl) piperazine-1-carboxamide was synthesized according to the synthesis method of item 1.7 in Example 1. The yield was 60%.

**Example 37. Preparation of *N*-(4-acetylphenyl)-4-(5-methyl-2-{[3-methoxy-4-(morpholin-4-yl)phenyl]amino)pyrimidin-4-yl)piperazine-1-carboxamide**

Using 2-methoxy-4-fluoronitrobenzene as a raw material, the key intermediate **E** was synthesized according to the synthesis method of items 1.1 to 1.2 in Example 1; using 5-methyl-2,4-dichloropyrimidine as a raw material, the key intermediate **G** was synthesized according to the synthesis method of items 1.3 to 1.5 in Example 1; using 4-aminoacetophenone as a raw material, the key intermediate **H** was synthesized according to the synthesis method of item 1.6 in Example 1, then *N*-(4-acetylphenyl)-4-(5-methyl-2-{[3-methoxy-4-(morpholin-4-yl)phenyl]amino}pyrimidin-4-yl) piperazine-1-carboxamide was synthesized according to the synthesis method of item 1.7 in Example 1. The yield was 60%.

The structures and **$^1$H-NMR and MS data** of the compounds of the examples are shown in Table 1.

**Table 1**

| Example | Structural formula | $^1$H-NMR, MS m/z data |
|---|---|---|
| 1 | 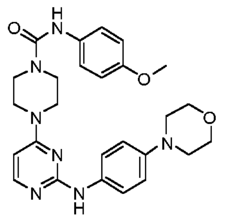 | $^1$H NMR (400 MHz, DMSO-*d*$_6$) δ 8.82 (s, 1H), 8.45 (s, 1H), 7.95 (d, *J* = 6.0 Hz, 1H), 7.56 (d, *J* = 9.0 Hz, 2H), 7.36 (d, *J* = 9.0 Hz, 2H), 6.87 - 6.82 (m, 4H), 6.24 (d, *J* = 6.1 Hz, 1H), 3.73 (t, *J* = 4.9 Hz, 4H), 3.71 (s, 3H), 3.64 - 3.63 (m, 4H), 3.55 - 3.53 (m, 4H), 3.01 (t, *J* = 4.7 Hz, 4H). MS (ESI) m/z: 490.46 [M+H]$^+$; |
| 2 | 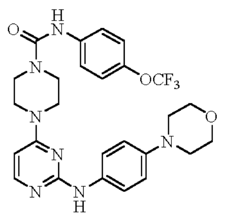 | $^1$H NMR (400 MHz, DMSO-*d*$_6$) δ 8.84 (s, 1H), 8.80 (s, 1H), 7.95 (d, *J* = 6.0 Hz, 1H), 7.59 - 7.55 (m, 4H), 7.25 (d, *J* = 8.6 Hz, 2H), 6.86 (d, *J* = 9.1 Hz, 2H), 6.25 (d, J = 6.1 Hz, 1H), 3.73 (t, *J* = 3.3 Hz, 4H), 3.65 (br, 4H), 3.57 - 3.56 (m, 4H), 3.01 (t, *J* = 3.3 Hz, 4H). MS (ESI) m/z: 544.64 [M+H]$^+$; |
| 3 | 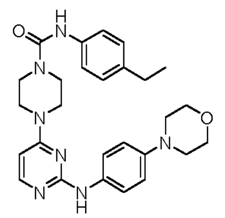 | $^1$H NMR (400 MHz, DMSO-*d*$_6$) δ 8.86 (s, 1H), 8.52 (s, 1H), 7.95 (d, *J* = 6.0 Hz, 1H), 7.56 (d, *J* = 9.0 Hz, 2H), 7.37 (d, *J* = 8.4 Hz, 2H), 7.08 (d, *J*= 8.4 Hz, 2H), 6.87 (d, *J*= 9.0 Hz, 2H), 6.25 (d, *J*= 6.1 Hz, 1H), 3.73 (t, *J*= 4.8 Hz, 4H), 3.64 (br, 4H), 3.55 - 3.54 (m, 4H), 3.01 (t, *J*= 4.7 Hz, 4H), 2.56 - 2.52 (m, 2H), 1.15 (t, *J* = 7.6 Hz, 3H). MS (ESI) m/z: 488.48 [M+H]$^+$; |
| 4 | 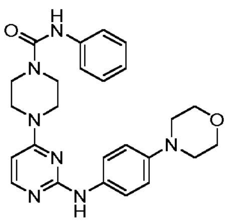 | $^1$H NMR (400 MHz, DMSO-*d*$_6$) δ 8.84 (s, 1H), 8.61 (s, 1H), 7.95 (d, *J* = 6.0 Hz, 1H), 7.56 (d, *J* = 9.0 Hz, 2H), 7.48 (d, *J*= 7.7 Hz, 2H), 7.24 (t, *J*= 7.9 Hz, 2H), 6.94 (t, *J* = 7.3 Hz, 1H), 6.86 (d, *J*= 9.0 Hz), 6.24 (d, *J* = 6.1 Hz, 1H), 3.81 - 3.69 (t, *J* = 4.8 Hz, 4H), 3.68 - 3.61 (m, 4H), 3.59 - 3.52 (m, 4H), 3.01 (t, *J* = 4.7 Hz, 4H). MS (ESI) m/z: 460.49 [M+H]$^+$. |

(continued)

| Example | Structural formula | $^1$H-NMR, MS m/z data |
|---|---|---|
| **5** | 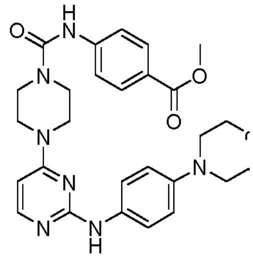 | $^1$H NMR (400 MHz, DMSO-$d_6$) δ 8.82 (s, 1H), 8.45 (s, 1H), 7.95 (d, J = 6.0 Hz, 1H), 7.56 (d, J = 9.0 Hz, 2H), 7.36 (d, J = 9.0 Hz, 2H), 6.85 (dd, J = 12.4, 9.0 Hz, 4H), 6.24 (d, J = 6.0 Hz, 1H), 3.75 - 3.71 (m, 4H), 3.71 (s, 3H), 3.68 - 3.59 (m, 4H), 3.58 - 3.49 (m, 4H), 3.04 - 2.98 (m, 4H). m.p.: 231.3 - 233.8 °C. MS (ESI) m/z: 490.5 $[M+H]^+$, 512.5 $[M+Na]^+$. |
| **6** | 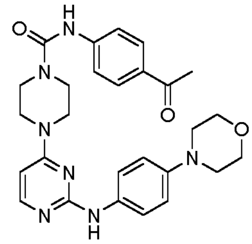 | $^1$H NMR (400 MHz, DMSO-$d_6$) δ 9.02 (s, 1H), 8.83 (s, 1H), 7.96 (d, *J* = 6.0 Hz, 1H), 7.88 (d, *J* = 8.8 Hz, 2H), 7.65 (d, *J* = 8.8 Hz, 2H), 7.57 (d, *J* = 9.0 Hz, 2H), 6.86 (d, *J* = 9.0 Hz, 2H), 6.24 (d, *J* = 6.1 Hz, 1H), 3.80 - 3.70 (m, 4H), 3.66 (m, 4H), 3.60 (m, *J* = 4.8 Hz, 4H), 3.35 (s, 3H), 3.06 - 2.97 (m, 4H). m.p.: 244.1 - 246.0 °C. MS (ESI) m/z: 502.5 $[M+H]^+$, 524.5 $[M+Na]^+$. |
| **7** | 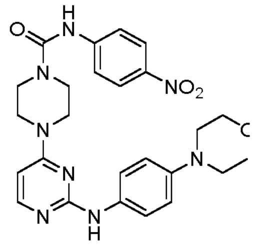 | $^1$H NMR (400 MHz, DMSO-$d_6$) δ 9.32 (s, 1H), 8.85 (s, 1H), 8.17 (d, *J* = 9.0 Hz, 2H), 7.96 (d, *J* = 5.9 Hz, 1H), 7.75 (d, *J* = 9.1 Hz, 2H), 7.56 (d, *J* = 8.7 Hz, 2H), 6.87 (d, *J* = 8.7 Hz, 2H), 6.25 (d, *J* = 6.0 Hz, 1H), 3.74 - 3.72 (m, 4H), 3.67 - 3.66 (m, 4H), 3.61 - 3.60 (m, 4H), 3.01 (t, *J* = 4.2 Hz, 4H). MS (ESI) m/z: 505.55 $[M+H]^+$. |
| **8** | 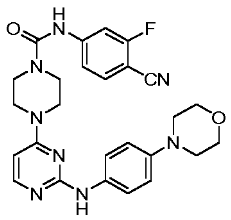 | $^1$H NMR (400 MHz, DMSO-$d_6$) δ 9.33 (s, 1H), 8.85 (s, 1H), 7.96 (d, *J* = 5.9 Hz, 1H), 7.78 - 7.71 (m, 2H), 7.56 (d, *J* = 8.7 Hz, 2H), 7.45 (d, *J* = 8.7 Hz, 1H), 6.86 (d, *J* = 8.8 Hz, 2H), 6.24 (d, *J* = 6.0 Hz, 1H), 3.74 - 3.72 (m, 4H), 3.66 (br, 4H), 3.59 - 3.57 (m, 4H), 3.01 (t, *J* = 4.2 Hz, 4H). MS (ESI) m/z: 503.47 $[M+H]^+$. |
| **9** | 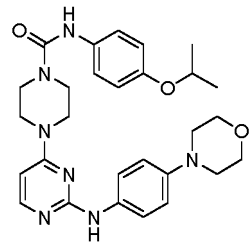 | $^1$H NMR (400 MHz, DMSO-$d_6$) δ 8.85 (s, 1H), 8.43 (s, 1H), 7.95 (d, *J* = 5.9 Hz, 1H), 7.56 (d, *J* = 8.7 Hz, 2H), 7.33 (d, *J* = 8.7 Hz, 2H), 6.86 (d, *J* = 8.8 Hz, 2H), 6.81 (d, *J* = 8.7 Hz, 2H), 6.25 (d, *J* = 6.0 Hz, 1H), 4.53 - 4.47 (m, 1H), 3.73 (t, *J* = 4.5 Hz, 4H), 3.64 (br, 4H), 3.53 (br, 4H), 3.01 (t, *J* = 4.2 Hz, 4H), 1.24 - 1.22 (m, 6H). MS (ESI) m/z: 518.25 $[M+H]^+$, 540.26 $[M+Na]^+$; |
| **10** | 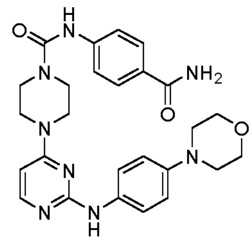 | $^1$H NMR (600 MHz, DMSO-$d_6$) δ 8.86 (s, 1H), 8.85 (s, 1H), 8.26 (q, *J* = 4.5 Hz, 1H), 7.96 (d, *J* = 6.1 Hz, 1H), 7.74 (d, *J* = 8.9 Hz, 1H), 7.57 (d, *J* = 2.8 Hz, 2H), 7.55 (d, *J* = 2.8 Hz, 2H), 6.87 (d, *J* = 9.0 Hz, 2H), 6.25 (d, *J* = 6.1 Hz, 2H), 3.74 - 3.72 (m, 4H), 3.65 (m, 4H), 3.58 - 3.56 (m, 4H), 3.03 - 3.00 (m, 4H), 2.76 (d, *J* = 4.5 Hz, 3H). MS (ESI) m/z: 503.5 $[M+H]^+$, 525.5 $[M+Na]^+$. |

(continued)

| Example | Structural formula | $^1$H-NMR, MS m/z data |
|---|---|---|
| **11** | 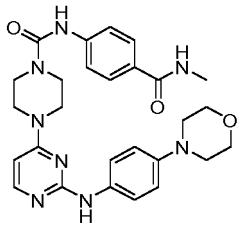 | $^1$H NMR (600 MHz, DMSO-$d_6$) δ 8.86 (s, 1H), 8.85 (s, 1H), 8.26 (q, *J* = 4.4 Hz, 1H), 7.96 (d, *J* = 6.0 Hz, 1H), 7.74 (d, *J* = 8.8 Hz, 2H), 7.57 (d, *J* = 2.9 Hz, 2H), 7.55 (d, *J* = 2.7 Hz, 2H), 6.87 (d, *J* = 9.1 Hz, 2H), 6.25 (d, *J* = 6.1 Hz, 1H), 3.75 - 3.71 (m, 4H), 3.65 (m, 4H), 3.57 (m, , 4H), 3.03 - 2.99 (m, 4H), 2.76 (d, *J* = 4.5 Hz, 3H). MS (ESI) m/z: 517.5 [M+H]$^+$, 539.5 [M+Na]$^+$. |
| **12** | 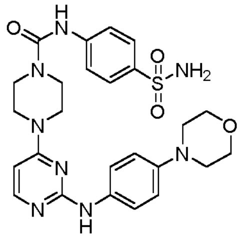 | $^1$H NMR (400 MHz, DMSO-$d_6$) δ 9.00 (s, 1H), 8.93 (s, 1H), 7.95 (d, *J* = 5.7 Hz, 1H), 7.70 (d, *J* = 8.6 Hz, 2H), 7.65 (d, *J* = 8.6 Hz, 2H), 7.55 (d, *J* = 8.5 Hz, 2H), 7.19 (s, 2H), 6.87 (d, *J* = 8.5 Hz, 2H), 6.27 (d, *J* = 5.8 Hz, 1H), 3.73 (m, 4H), 3.67 (m, 4H), 3.59 (m, 4H), 3.02 (m, 4H). MS (ESI) m/z: 539.4 [M+H]$^+$. |
| **13** | 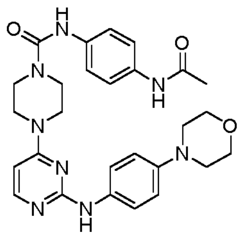 | MS (ESI) m/z: 517.3 [M+H]$^+$ |
| **14** | 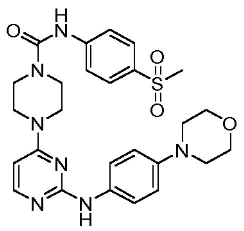 | $^1$H NMR (600 MHz, DMSO-$d_6$) δ 9.12 (s, 1H), 8.86 (s, 1H), 7.96 (d, *J* = 5.9 Hz, 1H), 7.79 (d, *J* = 8.8 Hz, 2H), 7.74 (d, *J* = 8.8 Hz, 2H), 7.56 (d, *J* = 8.8 Hz, 2H), 6.87 (d, *J* = 8.9 Hz, 2H), 6.25 (d, *J* = 6.0 Hz, 1H), 3.75 - 3.71 (m, 4H), 3.66 (s, 4H), 3.59 (m, 4H), 3.15 (s, 3H), 3.05-2.98 (m, 4H). MS (ESI) m/z: 538.6 [M+H]$^+$, 560.6 [M+Na]$^+$. |
| **15** | 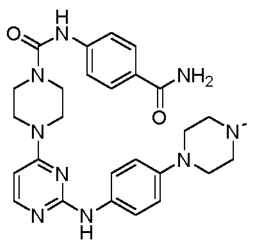 | $^1$H NMR (400 MHz, DMSO-$d_6$) δ 8.83 (d, *J* = 11.7 Hz, 2H), 7.95 (d, *J* = 5.9 Hz, 1H), 7.87 - 7.81 (m, 1H), 7.78 (d, *J* = 8.7 Hz, 2H), 7.58 - 7.52 (m, 4H), 7.16 (s, 1H), 6.85 (d, *J* = 8.7 Hz, 2H), 6.24 (d, *J* = 6.0 Hz, 1H), 3.62 (m, 8H), 3.04 (s, 4H), 2.46 (s, 4H), 2.22 (s, 3H). MS (ESI) m/z: 516.2 [M+H]$^+$. |
| **16** | 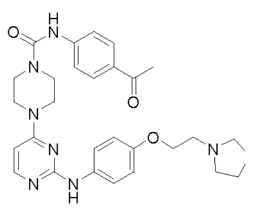 | $^1$H NMR (400 MHz, DMSO-$d_6$) δ 9.10 (s, 1H), 8.91 (s, 1H), 7.97 (d, *J* = 6.0 Hz, 1H), 7.87 (d, *J* = 8.6 Hz, 2H), 7.67 (d, *J* = 8.6 Hz, 2H), 7.60 (d, *J* = 8.8 Hz, 2H), 6.87 (d, *J* = 8.8 Hz, 2H), 6.27 (d, *J* = 6.0 Hz, 1H), 4.11 (t, *J* = 5.2 Hz, 2H), 3.65 (s, 4H), 3.60 (d, *J* = 4.0 Hz, 4H), 3.03 (s, 2H), 2.80 (s, 4H), 2.51 (s, 3H), 1.78 (s, 4H). MS (ESI) m/z: 530.2 [M+H]$^+$. |

(continued)

| Example | Structural formula | $^1$H-NMR, MS m/z data |
|---|---|---|
| **17** | 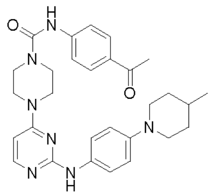 | $^1$H NMR (400 MHz, DMSO-$d_6$) δ 9.00 (s, 1H), 8.80 (s, 1H), 7.95 (d, *J* = 6.0 Hz, 1H), 7.88 (d, *J* = 8.6 Hz, 2H), 7.64 (d, *J* = 8.6 Hz, 2H), 7.52 (d, *J* = 8.8 Hz, 2H), 6.85 (d, *J* = 8.8 Hz, 2H), 6.23 (d, *J* = 6.0 Hz, 1H), 3.65 (s, 4H), 3.59 (s, 4H), 3.52 (d, *J* = 11.9 Hz, 2H), 2.55 (d, *J* = 11.6 Hz, 2H), 2.51 (s, 3H), 1.68 (d, *J*= 12.0 Hz, 2H), 1.5 1-1.39 (m, 1H), 1.27 - 1.18 (m, 2H), 0.94 (d, *J*= 6.4 Hz, 3H). MS (ESI) m/z: 514.2 $[M+H]^+$. |
| **18** | 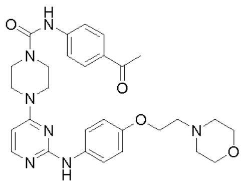 | $^1$H NMR (600 MHz, DMSO-$d_6$) δ 9.02 (s, 1H), 8.92 (s, 1H), 7.96 (d, *J* = 6.0 Hz, 1H), 7.88 (d, *J* = 8.8 Hz, 2H), 7.64 (d, *J*= 8.8 Hz, 2H), 7.59 (d, *J*= 9.0 Hz, 2H), 6.86 (d, *J* = 9.0 Hz, 2H), 6.27 (d, *J* = 6.0 Hz, 1H), 4.04 (t, *J* = 5.7 Hz, 2H), 3.66 (m, 4H), 3.59 (m, 8H), 3.35 (m, 2H), 2.71 (m, 2H), 2.51 (m, 5H). MS (ESI) m/z: 546.2 $[M+H]^+$, 568.3 $[M+Na]^+$. |
| **19** | 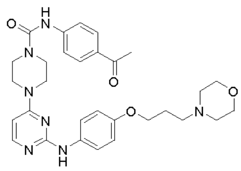 | $^1$H NMR (600 MHz, DMSO-$d_6$) δ 9.12 (s, 1H), 8.94 (s, 1H), 7.96 (d, *J* = 6.0 Hz, 1H), 7.88 (d, *J* = 8.8 Hz, 2H), 7.64 (d, *J*= 8.8 Hz, 2H), 7.59 (d, *J*= 9.0 Hz, 2H), 6.85 (d, *J*= 9.0 Hz, 2H), 6.27 (d, *J*= 6.0 Hz, 1H), 3.97 (t, *J*= 5.7 Hz, 2H), 3.67 (m, 8H), 3.60 (m, 4H), 3.35 (m, 2H), 2.68 (m, 2H), 2.51 (m, 5H), 1.96 (m, 2H). MS (ESI) m/z: 560.3 $[M+H]^+$, 582.2 $[M+Na]^+$. |
| **20** | 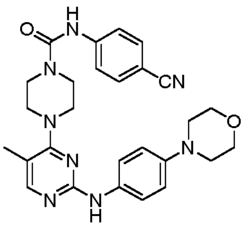 | $^1$H NMR (600 MHz, DMSO-$d_6$) δ 9.09 (s, 1H), 8.86 (s, 1H), 7.90 (s, 1H), 7.69 (s, 4H), 7.58 (d, *J* = 9.0 Hz, 2H), 6.86 (d, *J* = 9.0 Hz, 2H), 3.84 - 3.67 (m, 4H), 3.67 - 3.55 (m, 4H), 3.54 - 3.39 (m, 4H), 3.11 - 2.91 (m, 4H), 2.12 (s, 3H). $^{13}$C NMR (151 MHz, DMSO) δ 164.96, 159.23, 158.73, 154.84, 145.95, 145.65, 134.31, 133.33, 119.93, 119.45, 116.17, 107.90, 103.51, 66.67, 49.92, 47.43, 44.17, 16.61. LC-MS (ESI positive mode) m/z 499.09 ($[M+H]^+$); (ESI negative mode) m/z 497.17 ($[M-H]^-$) |
| **21** | 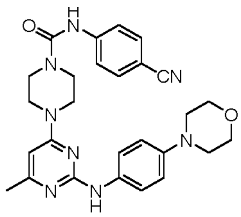 | $^1$H NMR (600 MHz, DMSO-$d_6$) δ 9.09 (s, 1H), 8.84 (s, 1H), 7.70 (s, 4H), 7.58 (d, *J*= 8.9 Hz, 2H), 6.86 (d, *J*= 9.0 Hz, 2H), 6.15 (s, 1H), 3.74 - 3.72 (m, 4H), 3.65 (s, 4H), 3.60 - 3.57 (m, 4H), 3.03 - 2.99 (m, 4H), 2.19 (s, 3H). $^{13}$C NMR (151 MHz, DMSO) δ 163.11, 159.68, 154.72, 146.03, 145.61, 134.21, 133.32, 120.28, 119.88, 119.47, 116.12, 103.54, 99.99, 93.43, 66.67, 56.49, 49.89, 43.86, 24.18, 19.02. MS (ESI) m/z: 499.4 $[M+H]^+$, 497.10 $[M-H]^-$ |
| **22** | 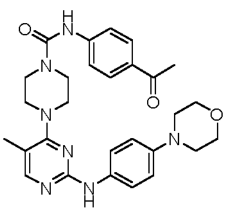 | $^1$H NMR (600 MHz, DMSO-$d_6$) δ 9.00 (s, 1H), 8.87 (s, 1H), 7.90 (s, 1H), 7.88 (d, *J*= 8.7 Hz, 2H), 7.65 (d, *J*= 8.7 Hz, 2H), 7.59 (d, *J*= 9.0 Hz, 2H), 6.86 (d, *J*= 9.0 Hz, 2H), 3.74 - 3.71 (m, 4H), 3.65 - 3.60 (m, 4H), 3.47 - 3.42 (m, 4H), 3.02 - 2.98 (m, 4H), 2.12 (s, 3H). $^{13}$C NMR (151 MHz, DMSO) δ 196.82, 164.96, 159.20, 158.71, 155.00, 145.95, 145.79, 134.30, 130.75, 129.68, 119.98, 118.66, 116.17, 107.89, 66.67, 49.92, 47.47, 44.18, 26.79, 16.62. MS (ESI) m/z: 516.11 $[M+H]^+$, 514.13 $[M-H]^-$ |
| **23** | 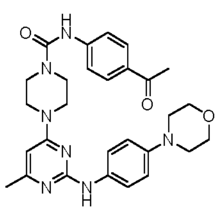 | $^1$H NMR (600 MHz, DMSO-$d_6$) δ 9.00 (s, 1H), 8.85 (s, 1H), 7.88 (d, *J* = 8.8 Hz, 2H), 7.65 (d, *J* = 8.8 Hz, 2H), 7.58 (d, *J*= 9.0 Hz, 2H), 6.86 (d, *J*= 9.0 Hz, 2H), 6.15 (s, 1H), 3.74 - 3.72 (m, 4H), 3.66 (s, 4H), 3.59 (dd, *J* = 6.1, 3.6 Hz, 4H), 3.02 -3.00 (m, 4H), 2.19 (s, 3H). $^{13}$C NMR (151 MHz, DMSO) δ 196.83, 165.88, 163.12, 159.66, 154.89, 146.04, 145.75, 134.20, 130.78, 129.68, 120.29, 118.67, 116.13, 93.44, 66.67, 49.89, 43.84, 26.79, 24.17. MS (ESI) m/z: 516.18$[M+H]^+$, 514.19 $[M-H]^-$ |

(continued)

| Example | Structural formula | $^1$H-NMR, MS m/z data |
|---|---|---|
| **24** | 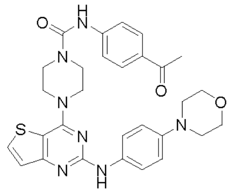 | $^1$H NMR (600 MHz, DMSO-$d_6$) δ 9.01 (s, 1H), 8.86 (s, 1H), 8.06 (d, *J*= 5.5 Hz, 1H), 7.89 (d, *J*= 8.7 Hz, 2H), 7.66 (d, *J*= 8.8 Hz, 2H), 7.64 (d, *J*= 8.9 Hz, 2H), 7.19 (d, *J*= 5.5 Hz, 1H), 6.89 (d, *J*= 9.0 Hz, 2H), 4.00 - 3.96 (m, 4H), 3.75 - 3.73 (m, 4H), 3.71 - 3.68 (m, 4H), 3.03 - 3.01 (m, 4H), 2.51 (s, 3H). $^{13}$C NMR (151 MHz, DMSO) δ 196.83, 163.69, 158.34, 158.19, 154.85, 146.01, 145.71, 134.47, 133.61, 130.81, 129.70, 124.15, 120.26, 118.68, 116.19, 105.99, 66.68, 49.94, 45.68, 43.91, 26.80. MS (ESI) m/z: 558.11 $[M+H]^+$, 556.12 $[M-H]^-$ |
| **25** | 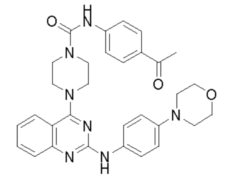 | $^1$H NMR (600 MHz, DMSO-$d_6$) δ 9.09 (s, 1H), 9.05 (s, 1H), 7.89 (s, 1H), 7.87 (d, *J*= 8.4 Hz, 2H), 7.75 (d, *J*= 8.0 Hz, 2H), 7.66 (d, *J*= 8.8 Hz, 2H), 7.62 (t, *J*= 7.7 Hz, 1H), 7.48 (d, *J*= 8.3 Hz, 1H), 7.19 (t, *J*= 7.4 Hz, 1H), 6.90 (d, *J* = 9.0 Hz, 2H), 3.76 - 3.71 (m, 13H), 3.06 - 3.02 (m, 4H), 2.51 (s, 3H). $^{13}$C NMR (151 MHz, DMSO) δ 196.84, 165.48, 156.42, 155.02, 146.29, 145.77, 134.05, 133.21, 130.78, 129.69, 125.93, 121.65, 120.47, 118.70, 116.10, 112.78 , 66.67, 49.84, 49.62, 44.01, 26.80. MS (ESI) m/z: 552.15 $[M+H]^+$, 510.17 $[M-H]^-$ |
| **26** | 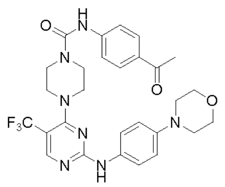 | $^1$H NMR (600 MHz, DMSO-$d_6$) δ 9.62 (s, 1H), 8.97 (s, 1H), 8.39 (s, 1H), 7.87 (d, *J*= 8.6 Hz, 2H), 7.64 (d, *J*= 8.6 Hz, 2H), 7.54 (d, *J*= 8.8 Hz, 2H), 6.91 (d, *J*= 8.7 Hz, 2H), 3.76 - 3.72 (m, 4H), 3.62 (d, *J* = 3.7 Hz, 8H), 3.08 - 3.03 (m, 4H), 2.51 (s, 3H). $^{13}$C NMR (151 MHz, DMSO) δ 196.83, 154.83, 147.20, 145.71, 132.26, 130.79, 129.68, 118.65, 115.85, 66.61, 65.38, 49.50, 47.8, 43.81, 26.80, 15.63. MS (ESI) m/z: 570.01$[M+H]^+$, 568.15 $[M-H]^-$ |
| **27** | 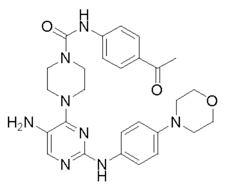 | $^1$H NMR (600 MHz, DMSO-$d_6$) δ 9.02 (s, 1H), 8.52 (s, 1H), 7.88 (d, *J*= 8.8 Hz, 2H), 7.73 (s, 1H), 7.65 (d, *J*= 8.8 Hz, 2H), 7.57 (d, *J*= 9.0 Hz, 2H), 6.84 (d, *J*= 9.1 Hz, 2H), 4.17 (s, 2H), 3.74 - 3.71 (m, 4H), 3.67 - 3.63 (m, 4H), 3.43 - 3.39 (m, 4H), 3.00 - 2.97 (m, 4H), 2.51 (s, 3H). $^{13}$C NMR (151 MHz, DMSO) δ 196.83, 155.81, 155.00, 153.73, 145.85, 145.26, 144.13, 135.32, 130.71, 129.67, 124.49, 118.91, 118.66, 116.38, 66.70, 50.14, 46.53, 44.11, 26.79. MS (ESI) m/z: 517.07$[M+H]^+$, 515.09 $[M-H]^-$ |
| **28** | 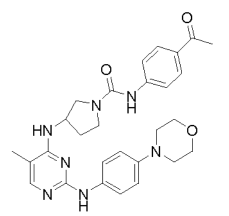 | $^1$H NMR (600 MHz, DMSO-$d_6$) δ 8.69 (s, 1H), 8.58 (s, 1H), 7.86 (d, *J* = 8.9 Hz, 2H), 7.71 (d, *J* = 8.9 Hz, 2H), 7.66 (s, 1H), 7.58 (d, *J* = 9.0 Hz, 2H), 6.82 (d, *J* = 9.0 Hz, 2H), 6.66 (d, *J* = 5.0 Hz, 1H), 3.91 - 3.87 (m, 1H), 3.69 - 3.66 (m, 4H), 3.64 - 3.61 (m, 1H), 3.51 - 3.47 (m, 1H), 3.37 (dd, *J* = 6.5, 3.7 Hz, 1H), 2.95 - 2.92 (m, 4H), 2.50 (s, 3H), 2.22 (d, *J* = 6.1 Hz, 1H), 2.13 - 2.05 (m, 2H), 1.94 (s, 3H). $^{13}$C NMR (151 MHz, DMSO) δ 196.80, 161.31, 153.88, 145.86, 130.55, 129.66, 120.02, 118.40, 116.15, 104.72, 66.63, 51.47, 49.89, 44.74, 26.77, 13.71. MS (ESI) m/z: 516.18$[M+H]^+$, 514.19 $[M-H]^-$ |
| **29** | 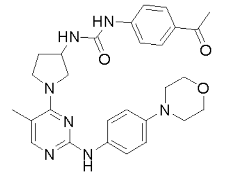 | $^1$H NMR (600 MHz, DMSO-$d_6$) δ 9.51 (s, 1H), 9.20 (s, 1H), 7.85 (d, *J* = 8.6 Hz, 2H), 7.66 (s, 1H), 7.52 (d, *J* = 8.6 Hz, 2H), 7.47 (d, *J* = 8.5 Hz, 2H), 7.08 (d, *J* = 6.5 Hz, 1H), 6.91 (d, *J* = 8.7 Hz, 2H), 4.29 (d, *J* = 4.9 Hz, 1H), 3.98 (dd, *J* = 11.0, 5.5 Hz, 1H), 3.92 - 3.83 (m, 2H), 3.75 - 3.70 (m, 4H), 3.67 (dd, *J* = 11.4, 3.1 Hz, 1H), 3.04 (s, 4H), 2.49 (s, 3H), 2.26 (s, 3H), 2.15 (t, *J* = 12.7 Hz, 1H), 1.91 (dd, *J* = 11.2, 5.7 Hz, 1H). $^{13}$C NMR (151 MHz, DMSO) δ 196.63, 161.26, 155.04, 145.43, 130.31, 130.09, 121.75, 116.96, 116.02, 66.59, 55.24, 53.86, 49.41, 49.12, 47.87, 31.03, 26.73, 17.29. MS (ESI) m/z: 516.18$[M+H]^+$, 514.19 $[M-H]^-$ |

(continued)

| Example | Structural formula | $^1$H-NMR, MS m/z data |
|---|---|---|
| **30** | 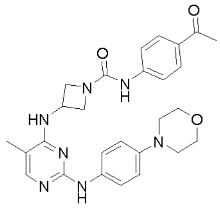 | $^1$H NMR (600 MHz, DMSO-$d_6$) δ 9.69 (s, 1H), 9.47 (s, 1H), 7.87 (s, 1H), 7.85 (d, *J*= 8.7 Hz, 2H), 7.54 (d, *J*= 8.7 Hz, 2H), 7.33 (d, *J* = 8.8 Hz, 2H), 7.16 (s, 1H), 6.93 (d, *J* = 9.0 Hz, 2H), 4.58 - 4.51 (m, 2H), 4.32 (t, *J* = 90 Hz, 1H), 3.75 - 3.72 (m, 4H), 3.54 - 3.50 (m, 1H), 3.41 (d, *J* = 14.0 Hz, 1H), 3.10 - 3.07 (m, 4H), 2.49 (s, 3H), 2.01 (s, 3H). $^{13}$C NMR (151 MHz, DMSO) δ 196.66, 157.75, 155.94, 149.48, 149.01, 145.45, 130.36, 130.07, 129.13, 125.15, 117.05, 115.45, 66.53, 50.40, 49.01, 42.71, 26.74, 12.44. MS (ESI) m/z: 502.10[M+H]$^+$, 500.18 [M-H]$^-$ |
| **31** | 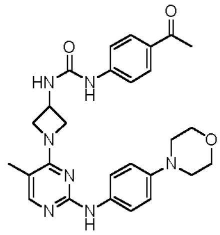 | $^1$H NMR (600 MHz, DMSO-$d_6$) δ 10.14 (s, 1H), 9.54 (s, 1H), 7.86 (d, *J*= 8.8 Hz, 2H), 7.62 (s, 1H), 7.57 (t, *J*= 7.7 Hz, 3H), 7.42 (d, *J*= 8.6 Hz, 2H), 6.96 (d, *J*= 9.0 Hz, 2H), 4.82 - 4.54 (m, 2H), 4.44 - 4.20 (m, 2H), 3.74 - 3.72 (m, 4H), 3.09 - 3.06 (m, 4H), 2.49 (s, 3H), 2.10 (s, 3H). $^{13}$C NMR (151 MHz, DMSO) δ 196.69, 161.58, 154.88, 145.41, 130.50, 130.04, 117.29, 115.94, 106.05, 66.55, 49.13, 40.78, 26.74, 14.26. MS (ESI) m/z: 502.10[M+H]$^+$, 500.11 [M-H]$^-$ |
| **32** | 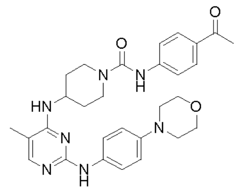 | MS (ESI) m/z: 530.20[M+H]÷, 528.21 [M-H]$^-$ |
| **33** | 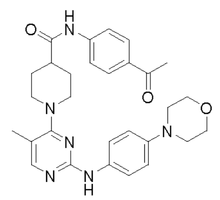 | $^1$H NMR (600 MHz, DMSO-$d_6$) δ 10.52 (s, 1H), 10.20 (s, 1H), 7.92 (d, *J*= 8.7 Hz, 2H), 7.77 (t, *J*= 6.8 Hz, 3H), 7.36 (d, *J*= 8.5 Hz, 2H), 7.00 (d, *J*= 8.1 Hz, 2H), 4.46 (d, *J*= 13.1 Hz, 2H), 3.75 - 3.72 (m, 4H), 3.24 (t, *J* = 12.0 Hz, 2H), 3.13 - 3.08 (m, 4H), 2.84 (td, *J* = 11.1, 5.5 Hz, 1H), 2.52 (s, 3H), 2.22 (s, 3H), 1.99 (d, *J* = 10.6 Hz, 2H), 1.74 (dd, J= 22.2, 10.5 Hz, 2H). $^{13}$C NMR (151 MHz, DMSO) δ 196.95, 173.75, 163.21, 144.10, 132.07, 129.89, 118.85, 116.03, 107.00, 66.47, 62.46, 49.11, 46.99, 42.50, 28.86, 26.88, 25.96, 18.28. MS (ESI) m/z: 515.15[M+H]$^+$, 513.23 [M-H]$^-$ |
| **34** | 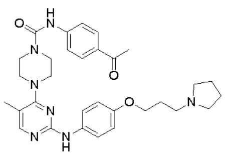 | MS (ESI) m/z: 558.3[M+H]$^+$ |
| **35** | 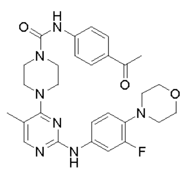 | MS (ESI) m/z: 534.2[M+H]$^+$ |
| **36** | 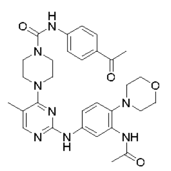 | MS (ESI) m/z: 573.3[M+H]$^+$ |

(continued)

| Example | Structural formula | $^1$H-NMR, MS m/z data |
|---|---|---|
| **37** | 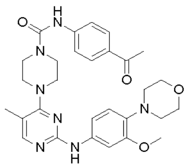 | MS (ESI) m/z: 546.3[M+H]$^+$ |

**The in vitro enzyme activity and cell anti-proliferation activity of the compounds of the present invention were studied, and the results were as follows**

The in vitro JAK2 and FLT3 kinase activity test was performed on the compounds of the 2-aminopyrimidine backbone of the above formula I (concentration 0.1 μM) according to the present invention using Mobility Shift Assay. The control compounds Pacritinib and Fedratinib were prepared by our research group.

The kinase buffer was composed of 50 mM HEPEs (pH 7.5) and 0.0015% BRIJ-35. The stop buffer contained a mixture of 100 mM HEPES (pH 7.5), 0.015% BRIJ-35, 0.2% coating reagent #3 and 50 mM EDTA. The compound to be tested was diluted with 100% DMSO to 50 times the highest concentration required in the reaction. The dilution of compound to be tested (100 μL) was transferred to the wells of a 96-well plate. 100 mL DMSO was added to two wells of the plate as a control group and the plate was marked as the source plate. 10 μL of compound was transferred from the source plate to a new 96-well plate to prepare an intermediate plate. In the intermediate plate, an additional 90 μL of kinase buffer was added to each well. The intermediate plate was shaken for 10 minutes, and then 5 μL of each well of the 96-well intermediate plate was copied to a 384-well plate as an analysis plate. The prepared enzyme solution was added to each well of the 384-well plate, then incubated at room temperature for 10 minutes, and then 10 μL of the prepared peptide solution (FAM-labeled peptide and ATP in kinase-based buffer) was added. The sample was incubated at 28°C for 1 hours, and then 25 μL of buffer was added. the conversion data from the Caliper program was copied and converted to the inhibition rate.

$$\text{Inhibition rate (\%)} = (\text{max-conversion}) / (\text{max-min}) \times 100.$$

Table 2 shows the in vitro kinase test results of JAK2 and FLT3 for some compounds.

**Table 2 Inhibitory activity of some compounds against JAK2 and FLT3 kinases**

| Compound | Percentage of inhibition against JAK2 at 0.1μM | Percentage of inhibition against FLT3 at 0.1μM |
|---|---|---|
| **Compound of Example 1** | 48.7 | 21.7 |
| **Compound of Example 2** | 54.3 | 30.5 |
| **Compound of Example 3** | 53.3 | 27.4 |
| **Compound of Example 4** | 53.7 | 30.8 |
| **Compound of Example 5** | 64.4 | 62.8 |
| **Compound of Example** 6 | 76.7 | 72.3 |
| **Compound of Example 7** | 79.3 | 84.4 |
| **Compound of Example 8** | 63.7 | 70.3 |

(continued)

| Compound | Percentage of inhibition against JAK2 at 0.1μM | Percentage of inhibition against FLT3 at 0.1μM |
|---|---|---|
| **Compound of Example 10** | 76.3 | 71.2 |
| **Compound of Example 11** | 68.1 | 35.8 |
| **Compound of Example 12** | 76.0 | 69.7 |
| **Compound of Example 13** | 73.0 | 70.8 |
| **Compound of Example 15** | 84.0 | 85.0 |
| **Compound of Example 16** | 45.0 | 69.0 |
| **Compound of Example 17** | 53.0 | 54.0 |
| **Compound of Example 18** | 61.7 | 70.6 |
| **Compound of Example 19** | 60.9 | 70.9 |
| **Compound of Example 20** | 88.5 | 95.2 |
| **Compound of Example 22** | 81.8 | 99.1 |
| **Compound of Example 24** | 44.0 | 68.0 |
| **Compound of Example 26** | 74.6 | 82.5 |
| **Compound of Example 27** | 22.8 | 67.9 |
| **Compound of Example 28** | 69.3 | 57.3 |
| **Compound of Example 29** | 57.4 | 75.7 |
| **Compound of Example 31** | 21.9 | 50.0 |
| **Compound of Example 32** | 80.5 | 78.6 |
| **Compound of Example** 33 | 74.3 | 97.0 |
| **Pacritinib** | 92.0 | 96.0 |
| **Fedratinib** | 95.2 | 88.7 |

The compound of the 2-aminopyrimidine skeleton of the above formula I according to the present invention was tested in vitro to inhibit the activity of human erythroleukemia cells HEL and human myeloid monocytic leukemia cells Molm-13. The control compounds Pacritinib and Fedratinib were prepared by our research group.

After the cells are resuscitated and passaged 2-3 times to stable state, 10 μL of the cell suspension was pipetted to a cell counting plate for counting, and the cell concentration was adjusted to $10\times10^4$ cells/mL. No cell solution was added to the outermost wells of the 96-well plate, and 100 μL of cell suspension was added to each of the remaining wells. 100 μL of medium was added to wells B2-B7 as a control group, and other wells were added with different concentrations of the compounds to be tested. After 72 hours of incubation in the incubator, 200μL of fresh medium was added to well A1 as a blank group, and then 20μL of CCK-8 solution was added to each well, and the incubation continued for 4 hours. The 96-well plate was shaken at room temperature for 3 minutes, and then the absorbance at 450 nm wavelength was measured with a microplate reader, and the inhibition rate of cell growth was calculated according to the following formula:

$$\text{Inhibition rate (\%)}=[(\text{A control - A addition}) / (\text{A control - A blank})]\times100$$

$IC_{50}$ is the drug concentration required to inhibit half of the cell growth. According to the results of drug concentration and inhibition rate, GraphPad Prism version 5 software is used to calculate $IC_{50}$ through the dose-effect curve.

Table 3 shows the test results of some compounds with human erythroleukemia cell HEL and human myeloid monocytic leukemia cell Molm-13 .

**Table 3 Results of anti-tumor cell proliferation activity of some compounds**

| Compound | HEL $IC_{50}$ (μM) | Molm-13 $IC_{50}$ (μM) |
|---|---|---|
| **Compound of Example 6** | 4.3 | 0.077 |
| **Compound of Example 20** | 0.9 | 0.072 |
| **Compound of Example 22** | 0.8 | 0.042 |
| **Compound of Example 26** | 4.6 | 0.083 |
| **Compound of Example 32** | 6.7 | 0.35 |
| **Compound of Example 33** | 1.7 | 0.062 |
| **Pacritinib** | 1.1 | 0.023 |
| **Fedratinib** | 0.89 | 0.068 |

Preliminary in vitro inhibitory kinase and tumor cell proliferation activity results show that the compounds of general formula I of the present invention have good inhibitory activities against JAK2 and FLT3, and some compounds are equal to or better than the positive control drugs Pacritinib and Fedratinib.

The specific embodiments described above further describe the purpose, technical solutions and beneficial effects of the present invention in further detail. It should be understood that the above descriptions are only specific embodiments of the present invention and are not intended to limit the present invention. Within the spirit and principle of the present invention, any modification, equivalent replacement, improvement, etc., shall be included in the protection scope of the present invention.

## Claims

1. A 2-aminopyrimidine compound of general formula I and its pharmaceutically acceptable salt, solvate or prodrug thereof,

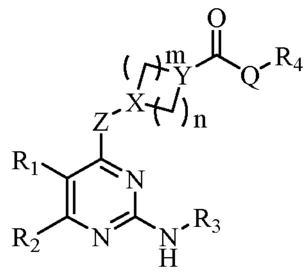

I

wherein, X and Y are the same or different, and are independently selected from N, CH and CHNH;
Q is NH or $CH_2$;
Z is selected from NH, $NCH_3$, O or a chemical bond;
m and n are the same or different, and are integers between 1 to 3;
$R_1$ and $R_2$ are the same or different, and are independently selected from the group consisting of hydrogen, halogen, $(C_1-C_6)$alkyl, $(C_1-C_6)$alkoxy, trifluoromethyl, cyano, amino, and nitro, or $R_1$ and $R_2$ together form $(C_6-C_{10})$ aryl, 5 to 10 membered heteroaryl or 4-10 membered heterocyclic group, the heterocyclic group optionally includes 0 to 3 double bonds;
$R_3$ is $(C_1-C_6)$ alkylacyl, $(C_6-C_{10})$ aryl or 5 to 10 membered heteroaryl, the aryl or heteroaryl is optionally substituted by 1 to 3 same or different $R_8$ groups;
Rs is hydrogen, hydroxy, halogen, cyano, $(C_1-C_6)$ alkyl, $(C_1-C_6)$ alkoxy, $(C_1-C_6)$ alkylacyl, $(C_1-C_6)$ carbamoyl, $-NR_5R_6$, $-(CH_2)_pNR_5R_6$, $-CONR_5R_6$, $-NHCONR_5R_6$, $-O(CH_2)_pNR_5R_6$, $-SO_2(CH_2)_pNR_5R_6$, $-SO_2(CH_2)_pCONR_5R_6$;
wherein $R_5$ and $R_6$ are the same or different, and are independently selected from the group consisting of hydrogen, $(C_1-C_6)$ alkyl, $(C_3-C_7)$ cycloalkyl, $(C_2-C_6)$ alkenyl, $(C_2-C_6)$ alkynyl, $(C_1-C_6)$ alkylacyl, and $(C_1-C_6)$ alkoxy;
or $R_5$ and $R_6$ together with the nitrogen atom to which they are connected form a 4 to 10 membered heterocyclic group or a 5 to 10 membered heteroaryl, the heterocyclic group or heteroaryl except for the nitrogen atom connected to $R_5$ and $R_6$, optionally contains 0 to 4 heteroatoms selected from N, O and/or S, the heterocyclic group optionally includes 0 to 3 double bonds, and the heterocyclic group or heteroaryl is optionally substituted by 0 to 3 same or different $R_7$ groups;
$R_7$ is $(C_1-C_6)$alkyl or $(C_3-C_7)$cycloalkyl;
p is an integer of 0 to 4;
$R_4$ is $(C_1-C_6)$ alkyl, $(C_3-C_7)$ cycloalkyl, $(C_6-C_{10})$ aryl, 5 to 10 membered heteroaryl, $(C_6-C_{10})$ arylmethyl, 5 to 10 membered heteroarylmethyl, the aryl or heteroaryl is optionally substituted by 1 to 3 same or different $R_9$ groups;
$R_9$ is hydrogen, hydroxy, halogen, halogenated $(C_1-C_6)$ alkyl, halogenated $(C_1-C_6)$ alkoxy, nitro, amino, cyano, $(C_1-C_6)$ alkyl, $(C_2-C_6)$ alkenyl , $(C_2-C_6)$ alkynyl, $(C_1-C_6)$ alkoxy, $(C_1-C_6)$ alkyl or $(C_1-C_6)$ alkoxy optionally substituted by hydroxy, amino or halogen, amino substituted by 1 to 2 $(C_1-C_6)$ alkyl, $(C_1-C_6)$ alkyl amide, free, salt-formed, esterified or amidated carboxyl, $(C_1-C_6)$ alkylsulfinyl, $(C_1-C_6)$ alkylsulfonyl, $(C_1-C_6)$ alkoxy, $(C_1-C_6)$ alkyl, $(C_1-C_6)$ alkylacyl, $(C_1-C_6)$ carbamoyl, carbamoyl substituted by 1 to 2 $(C_1-C_6)$ alkyl, $(C_1-C_3)$alkylenedioxy, or allyl.

**2.** The 2-aminopyrimidine compound of the general formula I and its pharmaceutically acceptable salt, solvate or prodrug thereof according to claim 1,

wherein, X and Y are the same or different, and are independently selected from N and CH;
Z is selected from NH, $NCH_3$ or a chemical bond;
$R_1$ and $R_2$ are the same or different, and are independently selected from hydrogen, halogen, $(C_1-C_6)$alkyl, $(C_1-C_6)$alkoxy, trifluoromethyl, cyano, amino, nitro, or $R_1$ and $R_2$ together form $(C_6-C_{10})$ aryl, or 5 to 10 membered heteroaryl;
$R_3$ is $(C_6-C_{10})$ aryl, 5 to 10 membered heteroaryl, and the aryl or heteroaryl is optionally substituted with 1 to 3 same or different $R_8$ groups.

**3.** The 2-aminopyrimidine compound of the general formula I and its pharmaceutically acceptable salt, solvate or prodrug thereof according to claim 2,

wherein,

m and n are the same or different and are 1 or 2;
$R_3$ is phenyl or 5 to 6 membered heteroaryl, and the phenyl or heteroaryl is optionally substituted with 1 to 3 same or different $R_8$ groups;
$R_8$ is $(C_1\text{-}C_6)$ alkoxy, $(C_1\text{-}C_6)$ alkylacyl, $(C_1\text{-}C_6)$ carbamoyl, $-NR_5R_6$, $-(CH_2)_pNR_5R_6$, $-CONR_5R_6$, $-NHCONR_5R_6$, $-O(CH_2)_pNR_5R_6$ or $-SO_2(CH_2)_pNR_5R_6$;
$R_5$ and $R_6$ are the same or different, and are independently selected from the group consisting of hydrogen, $(C_1\text{-}C_4)$alkyl, $(C_3\text{-}C_6)$cycloalkyl and $(C_1\text{-}C_4)$alkylacyl;
or $R_5$ and $R_6$ together with the nitrogen atom to which they are connected form

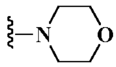 , 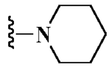 , 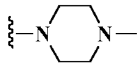 , 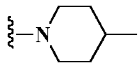 , 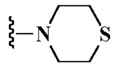 ,

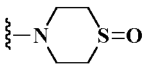 , 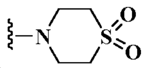 or 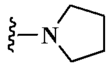 ;

$R_4$ is phenyl group, or 5-6 membered heteroaryl, and the phenyl or heteroaryl is optionally substituted with 1 to 3 same or different $R_9$ groups.

4. The 2-aminopyrimidine compound of general formula I and its pharmaceutically acceptable salt, solvate or prodrug thereof according to claim 3,

Z is NH or a chemical bond;
$R_1$ and $R_2$ are the same or different, and are independently selected from the group consisting of hydrogen, fluorine, chlorine, methyl, ethyl, cyclopropyl, methoxy, trifluoromethyl, cyano, amino, and nitro, or $R_1$ and $R_2$ together form phenyl or 5 to 6 membered heteroaryl;
Rs is $(C_1\text{-}C_6)$ alkoxy, $(C_1\text{-}C_6)$ alkylacyl, $(C_1\text{-}C_6)$ carbamoyl, $-NR_5R_6$, $-(CH_2)_pNR_5R_6$, $-CONR_5R_6$, $-NHCONR_5R_6$, or $-O(CH_2)_pNR_5R_6$;
wherein $R_5$ and $R_6$ are the same or different, and are independently selected from the group consisting of hydrogen, $(C_1\text{-}C_4)$alkyl and $(C_3\text{-}C_6)$cycloalkyl;
or $R_5$ and $R_6$ together with the nitrogen atom to which they are connected form

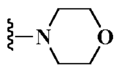 , 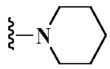 , 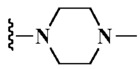 , 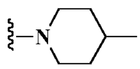 , 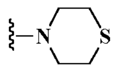 ,

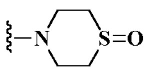 , 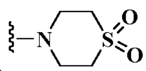 or 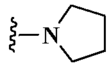 .

5. The 2-aminopyrimidine compound of general formula I and its pharmaceutically acceptable salt, solvate or prodrug thereof according to claim 4,

wherein,
Q is NH;
X and Y are N;
Z is a chemical bond;
m and n are 2;
$R_1$ and $R_2$ are the same or different, and are independently selected from the group consisting of hydrogen, fluorine, chlorine, methyl, methoxy, trifluoromethyl, amino, and nitro;
$R_3$ is phenyl optionally substituted with 1 to 3 same or different $R_8$ groups;
$R_8$ is $-NR_5R_6$, $-(CH_2)_pNR_5R_6$, $-CONR_5R_6$, or $-O(CH_2)_pNR_5R_6$;
p is an integer of 1 to 4;
$R_4$ is phenyl optionally substituted with 1 to 3 same or different $R_9$ groups.

6. The 2-aminopyrimidine compound of general formula I and its pharmaceutically acceptable salt, solvate or prodrug thereof according to claim 5,

wherein,
$R_2$ is hydrogen;
$R_8$ is $-NR_5R_6$, or $-O(CH_2)_pNR_5R_6$;
p is 2 or 3.

**7.** The 2-aminopyrimidine compound of general formula I and its pharmaceutically acceptable salt, solvate or prodrug thereof according to claim 6,

wherein,
$R_1$ is hydrogen, fluorine, chlorine, methyl or trifluoromethyl;
$R_5$ and $R_6$ together with the nitrogen atom to which they are connected form

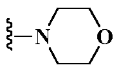, 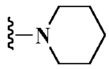, 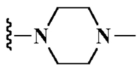, 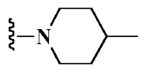, 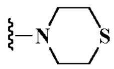, 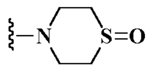,

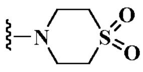 or 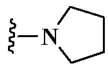.

**8.** The following 2-aminopyrimidine compounds of general formula I and their pharmaceutically acceptable salts, solvates or prodrugs thereof:

*N*-(4-methoxyphenyl)-4-(2-{[4-(morpholin-4-yl)phenyl]amino}pyrimidin-4-yl)piperazine-1-carboxamide;
*N*-(4-trifluoromethoxyphenyl)-4-(2-{[4-(morpholin-4-yl)phenyl]amino}pyri midin-4-yl)piperazine-1-carboxamide;
*N*-(4-ethylphenyl)-4-(2-{[4-(morpholin-4-yl)phenyl]amino}pyrimidin-4-yl)piperazine-1-carboxamide;
*N*-phenyl-4-(2-{[4-(morpholin-4-yl)phenyl]amino}pyrimidin-4-yl)piperazine -1-carboxamide;
*N*-(4-methoxyformylphenyl)-4-(2-{[4-(morpholin-4-yl)phenyl]amino}pyrimi din-4-yl)piperazine-1-carboxamide;
*N*-(4-acetylphenyl)-4-(2-{[4-(morpholin-4-yl)phenyl]amino}pyrimidin-4-yl)piperazine-1-carboxamide;
*N*-(4-nitrophenyl)-4-(2-{[4-(morpholin-4-yl)phenyl]amino}pyrimidin-4-yl)piperazine-1-carboxamide;
*N*-(4-cyano-3-fluorophenyl)-4-(2-{[4-(morpholin-4-yl)phenyl]amino}pyrimi din-4-yl)piperazine-1-carboxamide;
*N*-(4-isopropoxyphenyl)-4-(2-{[4-(morpholin-4-yl)phenyl]amino}pyrimidin-4-yl)piperazine-1-carboxamide;
*N*-(4-carbamoylphenyl)-4-(2-{[4-(morpholin-4-yl)phenyl]amino}pyrimidin-4-yl)piperazine-1-carboxamide;
*N*-(4-methylcarbamoylphenyl)-4-(2-{[4-(morpholin-4-yl)phenyl]amino}pyri midin-4-yl)piperazine-1-carboxamide;
*N*-(4-aminosulfonylphenyl)-4-(2-{[4-(morpholin-4-yl)phenyl]amino}pyrimid in-4-yl)piperazine-1-carboxamide;
*N*-(4-acetylaminophenyl)-4-(2-{[4-(morpholin-4-yl)phenyl]amino}pyrimidin -4-yl)piperazine-1-carboxamide;
*N*-(4-methanesulfonylphenyl)-4-(2-{[4-(morpholin-4-yl)phenyl]amino}pyri midin-4-yl)piperazine-1-carboxamide;
*N*-(4-carbamoylphenyl)-4-(2-{[4-(4-methylpiperazin-1-yl)phenyl]amino}pyri midin-4-yl)piperazine-1-carboxamide;
*N*-(4-acetylphenyl)-4-[2-({4-[2-(pyrrolidin-1-yl)ethoxy]phenyl}amino)pyrim idin-4-yl]piperazine-1-carboxamide;
*N*-(4-acetylphenyl)-4-(2-{[4-(4-methylpiperidin-1-yl)phenyl]amino}pyrimidi n-4-yl)piperazine-1-carboxamide;
*N*-(4-acetylphenyl)-4-[2-({4-[2-(morpholin-4-yl)ethoxy]phenyl}amino)pyrim idin-4-yl]piperazine-1-carboxamide;
*N*-(4-acetylphenyl)-4-[2-({4-[3-(morpholin-4-yl)propoxy]phenyl}amino) pyrimidin-4-yl]piperazine-1-carboxamide;
*N*-(4-cyanophenyl)-4-(5-methyl-2-{[4-(morpholin-4-yl)phenyl]amino} pyrimidin-4-yl)piperazine-1-carboxamide;
*N*-(4-cyanophenyl)-4-(6-methyl-2-{[4-(morpholin-4-yl)phenyl]amino} pyrimidin-4-yl)piperazine-1-carboxamide;
*N*-(4-acetylphenyl)-4-(5-methyl-2-{[4-(morpholin-4-yl)phenyl]amino} pyrimidin-4-yl)piperazine-1-carboxamide;
*N*-(4-acetylphenyl)-4-(6-methyl-2-{[4-(morpholin-4-yl)phenyl]amino} pyrimidin-4-yl)piperazine-1-carboxamide;
*N*-(4-acetylphenyl)-4-(2-{[4-(morpholin-4-yl)phenyl]amino}thieno[3,2-*d*]pyrimidin-4-yl)piperazine-1-carboxamide;
*N*-(4-acetylphenyl)-4-(2-{[4-(morpholin-4-yl)phenyl]amino}quinazolin-4-yl)piperazine-1-carboxamide;
*N*-(4-acetylphenyl)-4-(5-trifluoromethyl-2-{[4-(morpholin-4-yl)phenyl] amino}pyrimidin-4-yl)piperazine-1-carboxamide;
*N*-(4-acetylphenyl)-4-(5-amino-2-{[4-(morpholin-4-yl)phenyl]amino} pyrimidin-4-yl)piperazine-1-carboxamide;
*N*-(4-acetylphenyl)-3-[(5-methyl-2-{[4-(morpholin-4-yl)phenyl]amino} pyrimidin-4-yl)amino]pyrrolidine-1-carboxamide;

*N*-(4-acetylphenyl)-4-[(5-methyl-2-{[4-(morpholin-4-yl)phenyl]amino} pyrimidin-4-yl)amino]piperidine-1-carboxamide; and
*N*-(4-acetylphenyl)-4-[5-methyl-2-({4-[3-(pyrrolidin-1-yl)propoxy]phenyl} amino)pyrimidin-4-yl]piperazine-1-carboxamide.

**9.** The compound of general formula I according to claims 1 to 8, wherein the pharmaceutically acceptable salt thereof is a salt formed with an acid selected from: hydrochloric acid, hydrobromic acid, hydrofluoric acid, sulfuric acid, phosphoric acid, nitric acid, formic acid, acetic acid, propionic acid, oxalic acid, malonic acid, succinic acid, fumaric acid, maleic acid, lactic acid, malic acid, tartaric acid , citric acid, picric acid, methanesulfonic acid, ethanesulfonic acid, toluenesulfonic acid, benzenesulfonic acid, naphthalenesulfonic acid, trifluoroacetic acid and aspartic acid.

**10.** The compound of general formula I according to claim 9, wherein the salt formed with the acid is hydrochloride and methanesulfonate.

**11.** A pharmaceutical composition comprising the compound of general formula I according to any one of claims 1-10, and its pharmaceutically acceptable salt, solvate or prodrug thereof.

**12.** Use of the compound of any one of claims 1 to 10 and a pharmaceutically acceptable salt, hydrate, solvate or prodrug thereof in the preparation of a medicament for the prevention or treatment of diseases related to JAK2 kinase inhibitors.

**13.** Use of the compound of any one of claims 1 to 10 and a pharmaceutically acceptable salt, hydrate, solvate or prodrug thereof in the preparation of a medicament for the prevention or treatment of diseases related to FLT3 kinase inhibitors.

**14.** Use of the compound of any one of claims 1-10 and a pharmaceutically acceptable salt, hydrate, solvate or prodrug thereof in the preparation of a medicament for the prevention or treatment of diseases related to JAK2/FLT3 dual target kinase inhibitors.

**15.** Use of the compound of general formula I according to any one of claims 1-10, and a pharmaceutically acceptable salt, hydrate, solvate or prodrug thereof, or the composition according to claim 12 in the preparation of a medicament for the treatment of proliferative diseases and hematological malignancies.

**16.** The use according to claim 15, wherein the proliferative diseases comprise myelofibrosis, multiple myeloma, polycythemia vera, and primary thrombocythemia.

**17.** The use according to claim 15, wherein the hematological malignancies comprise acute myeloid leukemia and acute lymphocytic leukemia.

**INTERNATIONAL SEARCH REPORT**

International application No.
**PCT/CN2020/099683**

**A. CLASSIFICATION OF SUBJECT MATTER**

C07D 239/48(2006.01)i; C07D 401/14(2006.01)i; C07D 413/12(2006.01)i; A61K 31/5377(2006.01)i; A61K 31/541(2006.01)i; A61K 31/506(2006.01)i; A61P 35/00(2006.01)i; A61P 35/02(2006.01)i

According to International Patent Classification (IPC) or to both national classification and IPC

**B. FIELDS SEARCHED**

Minimum documentation searched (classification system followed by classification symbols)

C07D; A61P; A61K

Documentation searched other than minimum documentation to the extent that such documents are included in the fields searched

Electronic data base consulted during the international search (name of data base and, where practicable, search terms used)

CNABS, CNKI, CJFD, VEN, WOTXT, EPTXT, USTXT, 百度学术, BAIDU XUESHU, 超星读秀, SUPERSTAR DUXIU, 万方, WANFANG, 百度, BAIDU, CAPLUS, REGISTRY, MARPAT, WEB OF SCIENCE, 沈阳药科大学, 赵燕芳, 李颖修, 秦铭泽, 侯云雷, 韩雨霏, 田野, 叶天雨, 王鹏, 氨基嘧啶, 嘧啶, 吗啉, 哌嗪, 酪氨酸, 激酶, 抑制剂, JAK2, FLT3, 增殖, 增生, +piperazi+, +aminopyrimidine, +morpholi+, inhibitor+, janus kinases, tyrosine, rn号检索, rn number search: 2376308-!!-!, 2376309-!!-!, 2376310-!!-!, 2376312-!!-!, 2376319-!!-!, 2376320-!!-!, 2376322-!!-!, 2387611-!!-!, 2414943-!!-!, 结构式检索, structural formula search

**C. DOCUMENTS CONSIDERED TO BE RELEVANT**

| Category* | Citation of document, with indication, where appropriate, of the relevant passages | Relevant to claim No. |
|---|---|---|
| E | CN 111423384 A (SHENYANG PHARMACEUTICAL UNIVERSITY) 17 July 2020 (2020-07-17)<br>claims 1-10 | 1-17 |
| PX | CN 110317176 A (SHENYANG PHARMACEUTICAL UNIVERSITY) 11 October 2019 (2019-10-11)<br>tables 1 and 2, and claims 1-11 | 1-17 |
| PY | CN 110317176 A (SHENYANG PHARMACEUTICAL UNIVERSITY) 11 October 2019 (2019-10-11)<br>tables 1 and 2, and claims 1-11 | 1-17 |
| PX | LI, Yingxiu et al. "Discovery of 4-piperazinyl-2-aminopyrimidine Derivatives as Dual Inhibitors of JAK2 and FLT3"<br>*European Journal of Medicinal Chemistry,* Vol. 181, 07 August 2019 (2019-08-07), ISSN: 1768-3254,<br>p. 111590 | 1-17 |

☑ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **07 September 2020** | **13 October 2020** |

| Name and mailing address of the ISA/CN | Authorized officer |
|---|---|
| **China National Intellectual Property Administration (ISA/CN)**<br>**No. 6, Xitucheng Road, Jimenqiao, Haidian District, Beijing 100088**<br>**China** | |
| Facsimile No. **(86-10)62019451** | Telephone No. |

Form PCT/ISA/210 (second sheet) (January 2015)

**INTERNATIONAL SEARCH REPORT**

International application No.
**PCT/CN2020/099683**

**C. DOCUMENTS CONSIDERED TO BE RELEVANT**

| Category* | Citation of document, with indication, where appropriate, of the relevant passages | Relevant to claim No. |
|---|---|---|
| PY | CN 110862376 A (JIAXING TEKELUO BIOTECHNOLOGY CO., LTD.) 06 March 2020 (2020-03-06)<br>description, paragraphs [0006]-[0088] and [0523]-[0525] | 1-17 |
| X | "2060735-05-7"<br>*REGISTRY*, 29 January 2017 (2017-01-29), | 1-4 |
| X | "1794443-70-1"<br>*REGISTRY*, 05 July 2015 (2015-07-05), | 1-4 |
| X | "1794228-81-1"<br>*REGISTRY*, 05 July 2015 (2015-07-05), | 1-4 |
| X | "1794158-65-8"<br>*REGISTRY*, 05 July 2015 (2015-07-05), | 1-4 |
| X | "1794063-68-5"<br>*REGISTRY*, 05 July 2015 (2015-07-05), | 1-4 |
| X | "1794063-43-6"<br>*REGISTRY*, 05 July 2015 (2015-07-05), | 1-4 |
| X | "1794059-30-5"<br>*REGISTRY*, 05 July 2015 (2015-07-05), | 1-4 |
| X | "1793980-74-1"<br>*REGISTRY*, 05 July 2015 (2015-07-05), | 1-4 |
| X | "1793980-48-9"<br>*REGISTRY*, 05 July 2015 (2015-07-05), | 1-4 |
| X | CN 104540809 A (BLUEPRINT MEDICINES) 22 April 2015 (2015-04-22)<br>paragraph [0454] | 1-6 |
| A | CN 109776522 A (GUANGDONG HEC PHARMACEUTICAL CO., LTD. et al.) 21 May 2019 (2019-05-21)<br>entire document | 1-17 |
| A | CN 101679440 A (PALAU PHARMA SA) 24 March 2010 (2010-03-24)<br>entire document | 1-17 |
| A | CN 109311896 A (DAEWOONG PHARMACEUTICAL CO., LTD.) 05 February 2019 (2019-02-05)<br>entire document | 1-17 |

Form PCT/ISA/210 (second sheet) (January 2015)

**INTERNATIONAL SEARCH REPORT**
**Information on patent family members**

International application No.
**PCT/CN2020/099683**

| Patent document cited in search report | | | Publication date (day/month/year) | Patent family member(s) | | | Publication date (day/month/year) |
|---|---|---|---|---|---|---|---|
| CN | 111423384 | A | 17 July 2020 | None | | | |
| CN | 110317176 | A | 11 October 2019 | None | | | |
| CN | 110862376 | A | 06 March 2020 | None | | | |
| CN | 104540809 | A | 22 April 2015 | US | 2014378481 | A1 | 25 December 2014 |
| | | | | RU | 2015104342 | A | 27 August 2016 |
| | | | | AU | 2017272281 | A1 | 04 January 2018 |
| | | | | US | 10196436 | B2 | 05 February 2019 |
| | | | | MX | 2015000405 | A | 14 July 2015 |
| | | | | US | 9126951 | B2 | 08 September 2015 |
| | | | | NZ | 703495 | A | 23 February 2018 |
| | | | | CN | 104540809 | B | 11 December 2018 |
| | | | | US | 9340514 | B2 | 17 May 2016 |
| | | | | IL | 236611 | D0 | 26 February 2015 |
| | | | | AU | 2013290074 | A1 | 29 January 2015 |
| | | | | CA | 2878412 | A1 | 16 January 2014 |
| | | | | RU | 2679130 | C2 | 06 February 2019 |
| | | | | US | 2015210694 | A1 | 30 July 2015 |
| | | | | US | 2018362613 | A1 | 20 December 2018 |
| | | | | WO | 2014011900 | A2 | 16 January 2014 |
| | | | | JP | 2015523383 | A | 13 August 2015 |
| | | | | AU | 2017272281 | B2 | 04 July 2019 |
| | | | | MX | 369472 | B | 08 November 2019 |
| | | | | JP | 6104377 | B2 | 29 March 2017 |
| | | | | US | 2017066812 | A1 | 09 March 2017 |
| | | | | EP | 2872491 | A2 | 20 May 2015 |
| | | | | CN | 109627239 | A | 16 April 2019 |
| | | | | HK | 1206023 | A1 | 31 December 2015 |
| | | | | IL | 236611 | A | 26 February 2015 |
| | | | | US | 8802697 | B2 | 12 August 2014 |
| | | | | KR | 20150029030 | A | 17 March 2015 |
| | | | | US | 2014088100 | A1 | 27 March 2014 |
| | | | | ZA | 201500215 | B | 29 November 2017 |
| | | | | RU | 2019102203 | A | 05 March 2019 |
| | | | | SG | 11201500125Q | A | 27 February 2015 |
| | | | | US | 2019359682 | A1 | 28 November 2019 |
| | | | | BR | 112015000653 | A2 | 05 November 2019 |
| | | | | US | 2014187559 | A1 | 03 July 2014 |
| | | | | TW | 201429954 | A | 01 August 2014 |
| | | | | TW | I629266 | B | 11 July 2018 |
| | | | | UY | 35249 | A | 31 July 2014 |
| | | | | AR | 094312 | A1 | 22 July 2015 |
| CN | 109776522 | A | 21 May 2019 | None | | | |
| CN | 101679440 | A | 24 March 2010 | EP | 2142550 | A1 | 13 January 2010 |
| | | | | IL | 201073 | D0 | 17 May 2010 |
| | | | | KR | 20100015353 | A | 12 February 2010 |
| | | | | CA | 2682646 | A1 | 09 October 2008 |
| | | | | TW | 200904442 | A | 01 February 2009 |
| | | | | WO | 2008119792 | A1 | 09 October 2008 |
| | | | | MX | 2009010595 | A | 22 October 2009 |

Form PCT/ISA/210 (patent family annex) (January 2015)

**INTERNATIONAL SEARCH REPORT**
**Information on patent family members**

International application No.
**PCT/CN2020/099683**

| Patent document cited in search report | Publication date (day/month/year) | Patent family member(s) | Publication date (day/month/year) |
|---|---|---|---|
| | | US 2010113420 A1 | 06 May 2010 |
| | | CL 2008000946 A1 | 10 October 2008 |
| | | PE 20090996 A1 | 15 July 2009 |
| | | BR PI0809992 A2 | 14 October 2014 |
| | | JP 2010523522 A | 15 July 2010 |
| | | AR 065901 A1 | 08 July 2009 |
| | | US 2011160185 A9 | 30 June 2011 |
| | | AU 2008234822 A1 | 09 October 2008 |
| | | RU 2009140319 A | 10 May 2011 |
| CN 109311896 A | 05 February 2019 | EP 3478687 A1 | 08 May 2019 |
| | | CL 2018003511 A1 | 05 April 2019 |
| | | CO 2018013293 A2 | 14 December 2018 |
| | | WO 2018004306 A1 | 04 January 2018 |
| | | BR 112018074621 A2 | 06 March 2019 |
| | | AU 2017287762 B2 | 26 September 2019 |
| | | PE 20190811 A1 | 13 June 2019 |
| | | DO P2018000275 A | 31 October 2018 |
| | | SG 11201811470 P A | 30 January 2019 |
| | | PH 12018502507 A1 | 08 July 2019 |
| | | EC SP18093777 A | 31 December 2018 |
| | | JP 2019519579 A | 11 July 2019 |
| | | KR 20180003472 A | 09 January 2018 |
| | | AU 2017287762 C1 | 23 April 2020 |
| | | ZA 201808431 B | 27 May 2020 |
| | | EP 3478687 A4 | 19 February 2020 |
| | | CA 3025636 A1 | 04 January 2018 |
| | | IL 263306 D0 | 31 December 2018 |
| | | RU 2714206 C1 | 13 February 2020 |
| | | AU 2017287762 A1 | 13 December 2018 |
| | | MX 2018016287 A | 20 May 2019 |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 201910598383X


### Non-patent literature cited in the description


- *J. Med. Chem.,* 2011, vol. 54, 4638-4658
- *Cancer Cell,* 2008, vol. 13, 311-320
- *Blood,* 2004, vol. 103, 3669-3676